# EUROPEAN PATENT SPECIFICATION

(11) **EP 3 386 473 B1**
(45) Date of publication and mention of the grant of the patent: **20.04.2022**
(21) Application number: 16809352.4
(22) Date of filing: 07.12.2016
(51) Int. Cl.: A61K 8/41, A61Q 5/02, A61Q 5/12

(54) **OLIGOESTER AMMONIUM SALTS AND THEIR USE IN COSMETIC COMPOSITIONS**
OLIGOESTER AMMONIUMSALZE UND IHRE VERWENDUNG IN KOSMETISCHEN ZUSAMMENSETZUNGEN
SELS D'AMMONIUM D'OLIGOESTER ET LEUR UTILISATION DANS DES COMPOSITIONS COSMÉTIQUES

(30) Priority: 08.12.2015 EP 15198427; 03.08.2016 EP 16182521; 03.08.2016 EP 16182522; 23.08.2016 FR 1657858; 23.08.2016 FR 1657859; 09.11.2016 EP 16198041
(43) Date of publication of application: 17.10.2018
(73) Proprietor: Clariant International Ltd, 4132 Muttenz (CH)
(72) Inventor: KITA-TOKARCZYK, Katarzyna, 65812 Bad Soden (DE); BENSON, Hannah, 64625 Bensheim (DE); LEINWEBER, Dirk, 65719 Kelkheim (DE); PLATEN, Tobias, 65719 Hofheim am Taunus (DE); KRATZ, Anton, 65929 Frankfurt am Man (DE)
(74) Representative: Kampen, Daniela
(86) International application number: PCT/EP2016/080033
(87) International publication number: WO 2017/097819

(56) References cited:
- EP-A1- 1 136 471
- EP-A1- 1 160 238
- WO-A1-2015/091308
- WO-A1-2015/110269
- DE-C1- 19 715 835
- DE-C1- 19 743 687
- US-A- 5 880 299
- US-A1- 2003 130 162
- US-A1- 2008 214 776
- US-B1- 7 101 538

## Description

The present invention relates to cosmetic compositions, in particular hair conditioner compositions and shampoo compositions, comprising at least one oligoester ammonium salt, methods of preparing the cosmetic compositions, their use as well as methods of treating the hair.

Methods for cleaning and conditioning of hair are known for centuries. Frequently, the cleaning methods require an alkaline agent, such as soap. The interaction of this agent with hair results in weakening its mechanical properties and increases the porosity of hair fibers. Many known shampooing components result in dry hair that is damaged. Furthermore, the color of hair often fades with time due to washing and upon exposure to environmental factors such as sun and pollution. This can lead to dull appearance of the hair and results in more frequent washing than required. Frequent washing of hair may result in more damaged and less conditioned hair.

In order to improve the condition of hair and to keep a natural look of the hair over a longer period of time, improved hair care regimens and conditioning compositions are required. In particular the gloss (shine) of the hair and force needed for combing and detangling the hair are important characteristics.

Natural oils have been used for centuries to condition human hair. Essential oils (e.g. tea tree oil) and carrier oils (e.g. jojoba oil) have been used. Human hair contains about 97% of the protein keratin, which needs to be protected to preserve the strength and natural look.

The surface of keratin contains negatively charged amino acids. For this reason, hair conditioners can contain cationic components (e.g. surfactants) which are not washed out completely. The hydrophilic ends of the cationic components can bind to the keratin, whereas the hydrophobic ends of the molecules protect the hair surface.

Modern hair conditioning shampoos compositions are often intended to soften the hair, to improve the gloss of hair and to avoid a greasy look of the hair. Silicone derivatives, and quaternary ammonium compounds (surfactants or polymers) which coat the cuticle of the hair were used as conditioning ingredients in shampoo compositions. Such shampoos can be made transparent or opaque. Similarly, modern hair conditioner compositions have similar intentions. Fatty alcohols, silicone derivatives and quaternary ammonium compounds are typically used for hair conditioner compositions, which coat the cuticle of the hair.

Hair conditioner compositions can be used together with shampoo compositions or separately. Hair conditioner compositions comprise generally one or more of the following types of ingredients (components):
- acidity regulators, which maintain the conditioner's pH at about 3 to 5;
- antistatic agents;
- glossers, light-reflecting polymer components binding to the hair surface, such as silicones, e.g., dimethicone or cyclomethicone;
- lubricants, such as fatty alcohols or pro-vitamins, such as panthenol;
- moisturizers, which hold moisture in the hair and often contain humectants;
- oils (e.g. natural oils) for the hair to become soft and pliable;
- preservatives, to avoid microorganisms-growth in the composition;
- sequestrants, for improving the function of the composition in hard water;
- strengtheners, often containing hydrolyzed protein, to penetrate the hair and reinforce the structure, e.g. through polymer crosslinking;
- sun protectors against protein degradation and color loss, e.g. benzophenone; and
- surfactants, such as non-ionic surfactants, or ionic surfactants.

Hair shampoo compositions comprise generally one or more of the following types of ingredients (components):
- surfactants (ionic or non-ionic or amphoteric), with cleansing and foaming function polymers to thicken and stabilize the formulation. Salt can also be used to increase shampoo viscosity.
- conditioning agents (such as silicones, e.g. dimethicone or cyclomethicone, cationic polymers, cationic surfactants, etc.) making hair easier to detangle and more glossy
- actives such as anti-dandruff agents, e.g. piroctone olamine, zinc pyrithione, selenium sulfide, climbazole, etc.
- acidity regulators, which maintain the shampoo's pH at about 4 to 7;
- antistatic agents;
- lubricants, such as fatty alcohols or pro-vitamins, such as panthenol;
- moisturizers, which hold moisture in the hair and often contain humectants;
- oils (e.g. natural oils) for the hair to become soft and pliable;
- preservatives, to avoid microorganisms-growth in the composition;
- sequestrants, for improving the function of the composition in hard water;
- strengtheners, often containing hydrolyzed protein, to penetrate the hair and reinforce the structure, e.g. through polymer crosslinking;
- sun protectors against protein degradation and color loss, e.g. benzophenone.

Among many market trends, not only in cosmetics, it has been seen that ecological (also called green, natural or eco-friendly) products attract more consumers than before. To provide such products, ingredients are sought with good biodegradability, low irritation potential, non-allergenic, and with little impact on the environment. Often, such products are based on the (poly)ester chemistry, which can hydrolyze and degrade in aquatic environments. Esterquats are available from German manufacturers, such as Evonik or BASF.

DE-A 19710155 discloses a microemulsion comprising polyester ammonium salts of general formula (H), 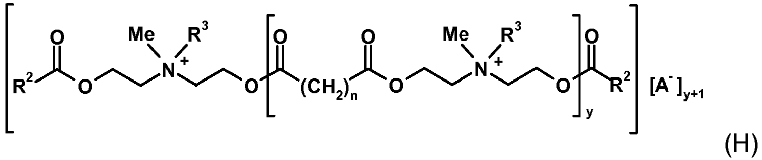 wherein
- R²: is C₁₁-C₂₁-alkyl,
- R³: is C₁-C₄-alkyl or hydroxyethyl,
- n: from 1 to 7,
- y: is either 0 or 1, and
- [A⁻]: is halogen or methylsulfate.

Therein a conditioning agent is proposed and the application of a micro-emulsion in hair conditioning is said to provide typical conditioning benefits, such as improved smoothness, softness and ease of combing wet hair.

DE-C 19743687 discloses a detergent composition comprising ester ammonium salts of general formula (K), 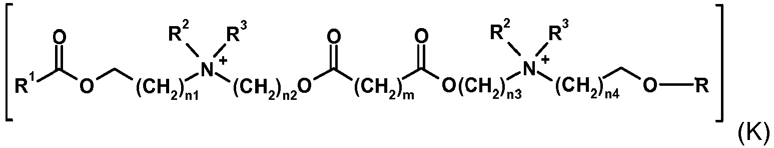 wherein
- R¹: is C₅-C₂₁-alkyl;
- R²: is C₁-C₄-alkyl;
- R³: is selected from C₁-C₄-alkyl or hydroxy-(C₁-C₄-alkyl);
- m: is from 1 to 10; and
- n1, n2, n3, n4: are from 0 to 4.

Therein it is further disclosed that the use of the composition in hair conditioning results in electrostatic charge reduction, ease of combing wet and dry hair, and improved softness. All of these effects are all typical for conditioning agents.

Hence, both the ester ammonium salts (H) and (K) are said to produce effects typical for conditioning agents. Nothing is to be found about other properties of this kind of compounds. US 7,101,538 describes a topical composition for skin, hair and nail treatment containing a polyester amine component. EP-A 1160238 discloses a process for preparing polyester quats. The application WO 2003/063790 describes various types of oligoester components for cosmetic applications. US 5,880,299 A discloses esterquats which may be made by reacting a mono- and a di-carboxylic acid with triethanolamine, then quaternizing. DE 197 15 835 C1 discloses esterquats which may be made by reacting a mono- and a di-carboxylic acid with diethanolamine, then quaternizing.

Shine, gloss and surface smoothening are all desirable attributes in cosmetic compositions and preparations for personal care. For example, shine and gloss are highly desired attributes in nail color and hair care preparations. Surface smoothening is important in shampoos, hair conditioners and other hair treatment preparations and can provide superior wet and dry hair combing. However, such attributes can often only be attained by combining several different ingredients in the cosmetic compositions. Thus, there is still a great need in personal care for new compositions and/or components that deliver multiple effects at the same time, such as several of the aforementioned attributes and benefits. In particular there is a great need for providing such compositions and/or components that provide excellent performance as a conditioner and a wide variety of cosmetic benefits simultaneously.

It is an objective of the present invention to provide cosmetic compositions, in particular hair conditioner compositions, comprising an ingredient that delivers at the same time multiple effects. The composition and its components can be prepared easily and provide or enhance several of the following benefits: hair detangling, improved wet and dry combing, shine (gloss) such as hair gloss without the need for silicone, conditioning, (hair) surface smoothening, hair repair, water resistance, film-forming properties, static charge reduction, anti-frizz, volume, thickening and surfactant activity. A further objective of the present invention to provide shampoo compositions comprising an ingredient that delivers at the same time multiple effects. Preferably, the composition and its components can be prepared easily and provide or enhance several of the following benefits: hair detangling, improved wet and dry combing, shine (gloss) such as hair gloss without the need for silicone, conditioning, (hair) surface smoothening, hair repair, water resistance, film-forming properties, static charge reduction, anti-frizz, volume, thickening and surfactant activity.

In a first aspect, the problems mentioned herein are solved by a cosmetic composition, in particular hair conditioner composition, comprising at least one oligoester ammonium salt (OAS) and at least one further cosmetically acceptable component (F), whereby the at least one oligoester ammonium salt (OAS) is obtainable by the following steps:
(i) heating a mixture of the following components (a) to (d) under continuously removing of reaction water:
   0.5 to 3.0 molar equivalents, preferably 0.6 to 2.0 molar equivalents, more preferably 0.75 to 1.5 molar equivalents, of a diethanolamine compound represented by the formula (a) 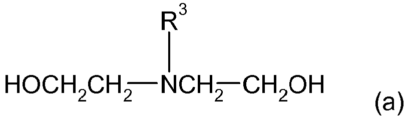 wherein R³ is a linear or branched C₁-C₆-alkyl, preferably linear or branched C₁-C₄-alkyl, more preferably methyl or ethyl;
   0.5 to 1.5 molar equivalents of a dicarboxylic acid represented by the formula (b) 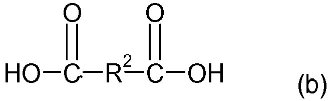 wherein R² is a linear or branched C₁-C₁₀-alkylene or a linear or branched C₂-C₁₀-alkenylene, preferably C₂-C₈-alkylene, more preferably C₄-alkylene;
   0.5 to 1.5 molar equivalents of an organic triol represented by the formula (c) 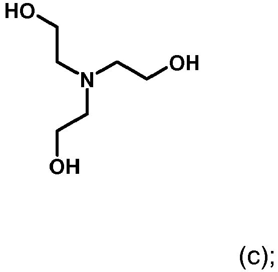
   1.0 molar equivalent of a monocarboxylic acid represented by formula (d)

      R¹-COOH (d)
   wherein R¹ is linear or branched C₁₂-C₂₄-alkyl or linear or branched C12-C24-alkenyl, preferably linear or branched C₁₂-C₂₂-alkyl or linear or branched C₁₂-C₂₂-alkenyl, more preferably C₂₀-C₂₂-alkyl;
(ii) reacting the oligoester product of step (i) with a quaternization agent (e), in particular from the group of dimethyl sulfate, diethyl sulfate and alkyl halides;
(iii) optionally removing and purifying the at least one oligoester ammonium salt.

The OAS (and the cosmetic composition) is preferably prepared where the di-ethanolamine component (a) wherein R³ is methyl.

The OAS (and cosmetic composition) is preferably prepared wherein the dicarboxylic acid (b) is selected from the group consisting of adipic acid, glutaric acid, succinic acid, sebacic acid, itaconic acid, maleic acid, and combinations thereof.

The OAS (and cosmetic composition) is prepared wherein the organic triol (c) is triethanolamine.

The OAS (and cosmetic composition) is preferably prepared by choosing the carboxylic acid (d) selected from the group consisting of behenic acid, oleic acid, coconut fatty acid, linoleic acid, and combinations thereof.

The OAS (and cosmetic composition) is preferably prepared by choosing the components (a), (b), (c) and (d) in the following molar ratios: from 0.5 - 3.0 (a), from 0.5 - 1.5 (b), from 0.5 - 1.5 (c) and 1 (d). The component (a) can also preferably be used in a molar ratio of 0.75 - 3.0.

The OAS (and cosmetic composition) is preferably prepared by choosing the molar ratio of the components (a), (b), (c) and (d) such that the molar equivalents of hydroxyl functions are in excess of the molar equivalents of the acid functions.

The OAS (and cosmetic composition) is preferably prepared by choosing a process, whereby first heating the mixture of components (a), (b), (c) and (d) in step (i) to a temperature from 80 to 220°C, preferably from 150 to 210°C, more preferably from 160 to 200°C is used, and in step (ii) subsequent reaction of the obtained oligoester product occurs with a quaternization agent (e), preferably selected from the group consisting of dimethyl sulfate, diethyl sulfate, methyl chloride, ethyl chloride, butyl chloride, and combinations thereof.

The cosmetic composition is preferably prepared wherein the oligoester ammonium salt (OAS) having a molecular mass Mn (number average) of from 500 to 4000 g/mol, preferably from 600 to 4000 g/mol, more preferably from 650 to 2000 g/mol, more preferably from 800 to 1900 g/mol, even more preferably from 665 to 1800 g/mol, even more preferably from 900 to 1800 g/mol.

In a particularly preferred embodiment of the first aspect, the components (a) to (c) are: methyl diethanol amine (component (a)), behenic acid (component (d)), adipic acid or sebacic acid (component (b)), and triethanolamine (component (c)), respectively.

In a second aspect, the invention also relates to a cosmetic composition, in particular hair conditioner composition or a shampoo composition, comprising at least one oligoester ammonium salt (OAS) of formula (I) and at least one further cosmetically acceptable component (F), 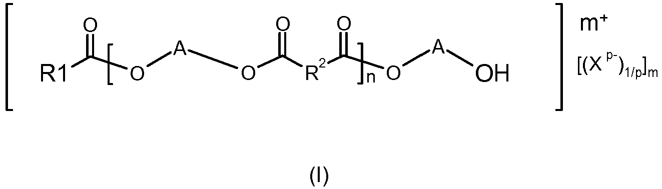 wherein:
- R¹: is a linear or branched C₂₀-C₂₄-alkyl or linear or branched C₂₀-C₂₄-alkenyl group, preferably a linear or branched C₂₀-C₂₂-alkyl group;
- R²: is a linear or branched C₁-C₈-alkylene or linear or branched C₂-C₈-alkenylene group;
- A: is, at least once, independently selected from quaternary ammonium compounds of the formula (II) 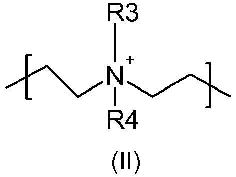 wherein R³, R⁴ are each independently linear or branched C₁-C₆-alkyl groups;
and A is also, at least once, independently selected from quaternary ammonium compounds of the formula (III) 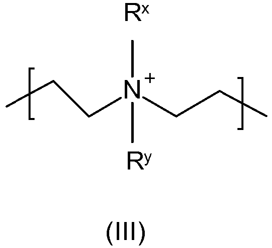 wherein R^{x} is a linear or branched hydroxy-C₁-C₆-alkyl group and R^{y} is a linear or branched C₁-C₆-alkyl;
- n: is an integer from 1 to 30, preferably from 1 to 10; and
- m: is an integer from 1 to 31, describing the charge, preferably m is an integer from 1 to 10;
- [(X^{p-})_{1/p}]: is a counter-ion selected from the group consisting of monovalent anions having the formula [X⁻], divalent anions having the formula [(X²⁻)_{0.5}], and trivalent anions having the formula [(X³⁻)_{1/3}].

The phrase "A is, at least once, independently selected from" means that the compound conforming to formula (I) must contain, at position A, a compound conforming to the requirements thereafter described. Indeed, since n is an integer from 1 to 30, not all of the repeating units described in the square brackets for n need be exactly the same chemically. Hence, where n is 3, then A described in the square brackets for n could be three different chemical groups or the same chemical group. The phrase "A is, at least once, independently selected from" means that the compound conforming to formula (I) must contain, at position A, at least once a compound conforming to the requirements thereafter described - where n is 3, then A could be said compound for all repeating units, or only for one occurrence of A - the minimum is a least once.

In at least one embodiment of the cosmetic composition, wherein in formula (I) the counter ion of the formula [(X^{p-})_{1/p}] is selected from the group consisting of Cl-(chloride), Br⁻ (bromide), MeSO₄⁻ (methyl sulfate), EtSO₄⁻ (ethyl sulfate), HCO₂⁻(formate), citrate, acetate, nitrate, [(CO₃²⁻)_{0.5}] (carbonate), [(SO₄²⁻)_{0.5}] (sulfate), [(PO₄³⁻)_{1/3}] (phosphate), and combinations thereof.

In at least one embodiment the cosmetic composition, comprises from 0.01 to 20% by weight of one or more oligoester ammonium salts (OAS) and further comprises at least 0.5% by weight of one or more further components (F) selected from the group consisting of acidity regulators, glossers, lubricants, and further surfactants.

In at least one embodiment the composition preferably comprises from 0.01 to 20% by weight of one or more OAS and at least 0.5% by weight of further surfactant(s), preferably cetrimonium chloride (CTAC).

A third aspect relates to the use of an oligoester ammonium salt (OAS) obtained by a process as defined above, or of an oligoester ammonium salt (OAS) of formula (I) as a surfactant in cosmetic compositions, preferably in hair conditioner compositions or in hair shampoo compositions.

A fourth aspect relates to a method of preparing a cosmetic composition, in particular a hair conditioner composition or a hair shampoo composition, comprising the step of preparing one or more oligoester ammonium salts (OAS) as described above, and mixing the OAS with one or more further components (F) and water.

The hair conditioning compositions can be prepared by any conventional method well known in the art. For example, they can be prepared as follows: deionized water is heated to 85°C. The oligoester ammonium salts (optionally purified) and one or more further components (F) are mixed into the water. The water is maintained at a temperature of about 85°C until the components are homogenized, and no solids are observed. The mixture is then cooled to about 55°C and maintained at this temperature. After it is homogenized, it is cooled to room temperature (20°C).

A fifth aspect relates to a method of treating hair, comprising:
a) applying the hair conditioner composition according to the first aspect or the second aspect onto wet hair and then
b) removing the conditioner composition from the hair.

An alternative embodiment of the fifth aspect relates to a method of treating hair, comprising:
a) applying the hair shampoo composition according to the first aspect or the second aspect onto wet hair and then
b) removing the shampoo composition from the hair.

At least one embodiment of the fifth aspect relates to a method of treating hair, comprising:
(a) applying a shampoo composition onto the hair; and then
(b) washing the hair with the shampoo composition; and then
(c) removing the shampoo composition from the hair; and then
(d) applying the hair conditioner composition onto wet hair, and then removing the conditioner composition from the hair.

In an preferred embodiment of the fifth aspect, either the shampoo composition or the conditioner composition, or both compositions, are according to the first aspect or the second aspect.

The hair conditioner composition as defined above can either be used on precleaned hair or without pre-treatment. Preferably, the hair is being washed with shampoo prior to the application of the conditioner composition.

The method of treating hair according to the present invention may also include one or more additional steps using commonly known compositions like, for example, a color altering composition, a developer composition, a pre-treatment composition and/or a post-treatment composition. Such commonly known compositions include well-known conventional additives being typically applied in hair treatment compositions, such as coloring agents, basifying and acidifying agents, buffers, thickening agents, gelling agents, rheological modifiers, antioxidants, fragrances and chelating agents.

The preferred features and components of the present invention are hereinafter described in more detail.

In the context of the present invention, the expression "C₁-C₂₄-alkyl" includes all kind of acyclic and cyclic alkyl groups with 1 to 24 carbon atoms. In at least one embodiment, the compounds conforming to C₁-C₂₄-alkyl are selected from the group consisting of methyl, ethyl, n-propyl, iso-propyl, n-butyl, sec-butyl, iso-butyl, tert-butyl, n-pentyl, 1-methylbutyl, 2-methylbutyl, 3-methylbutyl, 1,1-dimethylpropyl, 2,2-dimethylpropyl, 1,2-dimethylpropyl, 1-ethylpropyl, n-hexyl, 1-methylpentyl, 2-methylpentyl, 3-methylpentyl, 4-methylpentyl, 1,1,-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1-ethyl-1-methyl-propyl, 1-ethyl-2-methylpropyl, 2-ethyl-1-methyl-propyl und 2-ethyl-2-methylpropyl, n-heptyl, 5-methylhexyl, 2-ethyl-3-methylbutyl, n-octyl, 5-methylheptyl, 4,4-dimethylhexyl, 3-ethylhexyl, 2-ethyl-3-methylpentyl, nonyl, decyl, undecyl, dodecyl, tridecyl, tetradecyl, pentadecyl, hexadecyl, heptadecyl, octadecyl, nonadecyl, eicosanyl, heneicosanyl, docosanyl, tricosanyl, tetracosanyl, as well as cyclopropyl, cyclobutyl, cyclopentyl, 1-metylcyclobutyl, 2-methylcyclobutyl, 3-methylcyclo-butyl, cyclohexyl, 1-methyl-cyclopentyl, 2-methylcyclopentyl, 3-methylcyclopentyl, 1,2-dimethylcyclobutyl, 1,3-dimethyl-cyclobutyl, 2,2-dimethylcyclobutyl, 2,3-dimethylcyclobutyl und 3,3-dimethylcyclobutyl, cyclo-decyl, decalinyl, and mixtures thereof.

In the context of the present invention, the expression "C₂-C₂₄-alkenyl" includes all kind of acyclic and cyclic hydrocarbon groups with 2 to 24 carbon atoms and one or more C-C double bonds. In at least one embodiment, the compounds conforming to C₂-C₂₄-alkenyl are selected from the group consisting of vinyl, prop-1-enyl, prop-2-enyl, methallyl, 1-methylallyl, homoallyl, but-2-enyl, pent-1-enyl, pent-2-enyl, pent-3-enyl, 1-methylbut-1-enyl, 2-methylbut-1-enyl, 3-methylbut-1-enyl, 1-methylbut-2-enyl, 2-methylbut-2-enyl, 3-methylbut-2-enyl, 1-methyl-but-3-enyl, 2-methylbut-3-enyl, 3-methylbut-3-enyl, 1-ethylprop-1-enyl, 1-ethyl-prop-2-enyl, hex-1-enyl, hex-2-enyl, hex-3-enyl, hex-4-enyl, hex-5-enyl,1-methylpent-1-enyl, 2-methylpent-1-enyl, 3-methylpent-1-enyl, 4-methylpent-1-enyl, 1-methylpent-2-enyl, 2-methylpent-2-enyl, 3-methylpent-2-enyl, 4-methylpent-2-enyl, 1-methylpent-3-enyl, 2-methylpent-3-enyl, 3-methylpent-3-enyl, 4-methylpent-3-enyl, 1-methylpent-4-enyl, 2-methylpent-4-enyl, 3-methylpent-4-enyl, 4-methylpent-4-enyl, 1,2-dimethylbut-1-enyl, 1,3-dimethylbut-1-enyl, 3,3-dimethylbut-1-enyl, 1,1-dimethylbut-2-enyl, 1,2-dimethylbut-2-enyl, 1,3-dimethylbut-2-enyl, 2,3-dimethylbut-2-enyl, 1,1-dimethylbut-3-enyl, 1,2-dimethylbut-3-enyl, 1,3-dimethylbut-3-enyl, 2,2-dimethylbut-3-enyl and 2,3-dimethylbut-3-enyl, heptenyl, octenyl, nonenyl, decenyl, undecenyl, dodecenyl, tridecenyl, tetradecenyl, pentadecencyl, hexadecenyl, heptadecenyl, octadecenyl, nonadecenyl, eicosenyl, heneicosenyl, docosenyl, tricosenyl, tetracosenyl, and cyclobut-1-enyl, cyclobut-2-enyl, cyclopent-1-enyl, cyclopent-2-enyl, cyclopent-3-enyl, cyclohex-1-enyl, cyclohex-2-enyl, cyclohex-3-enyl, cyclodecenyl, and mixtures thereof.

Within the context of the present invention the expression "C₁-C₁₀-alkylene" includes all kind of acyclic, saturated hydrocarbon units having the formula CₘH₂ₘ, all kind of monocyclic, saturated hydrocarbon units having the formula CₘH₂ₘ₋₂ and all kind of bicyclic, saturated hydrocarbon units having the formula CₘH₂ₘ₋₄, wherein m in said formulae is an integer selected from 1 to 10, where possible. In at least one embodiment, the compounds conforming to C₁-C₁₀-alkylene are selected from the group consisting of: methylene, ethylene, n-propylene, 1-methylethylene, 2-methylethylene, butylene, 1-methylpropylene, butylene, 2-methyl-propylene, 3-methylproylene, 1,1-dimethylethylene, 1,2-dimethylethylene, pentylene, 1-methylbutylene, 2-methylbutylene, 3-methylbutylene, 1,1-dimethylpropylene, 2,2-dimethyl-propylene, 1,2-dimethylpropylene, 1-ethylpropylene, hexylene, 1-methylpentylene, 2-methylpentylene, 3-methylpentylene, 4-methylpentylene, 1,1,-dimethylbutylene, 1,2-dimethylbutylene, 1,3-dimethylbutylene, 2,2-dimethylbutylene, 2,3-dimethylbutylene, 3,3-dimethylbutylene, 1-ethylbutylene, 2-ethylbutylene, 1-ethyl-1-methyl-propylene, 1-ethyl-2-methylpropylene, 2-ethyl-1-methyl-propylene, 2-ethyl-2-methyl-propylene, heptylene, 5-methylhexylene, 2-ethyl-3-methylbutylene, octylene, 5-methyl-heptylene, 4,4-dimethyl-hexylene, 3-ethylhexylene, 2-ethyl-3-methylpentylene, nonylene and decylene as well as 1,3-cyclopentylene, 1,3-cyclohexylene, decalinylene, and mixtures thereof.

In the context of the present invention, the expression "C₂-C₁₀-alkenylene" includes all kind of acyclic, mono-unsaturated hydrocarbon units having the formula CₘH₂ₘ₋₂, all kind of acyclic di- and poly-unsaturated hydrocarbon units having the formulae CₘH₂ₘ₋₄, CₘH₂ₘ₋₆ etc., as well as cyclic, mono- and diunsaturated hydrocarbon units having the formulae CₘH₂ₘ₋₄, CₘH₂ₘ₋₆ etc., wherein m in said formulae is an integer selected from 2 to 10, where possible. In at least one embodiment, the compounds conforming to C₂-C₁₀-alkenylene are selected from the group consisting of: ethenylene, prop-1-enylene, prop-2-enylene, but-1-enylene, but-2-enylene, but-3-enylene, pent-1-enylene, pent-2-enylene, pent-3-enylene, pent-4-enylene, pent-5-enylene, 1-methylbut-1-enylene, 1-methylbut-2-enylene, 1-methylbut-3-enylene, hex-1-enylene, hex-2-enylene, hex-3-enylene, hept-1-enylene, hept-2-enylene, hept-3-enylene, oct-1-enylene, oct-2-enylene, oct-3-enylene, oct-4-enylene, non-1-enylene, non-2-enylene, non-3-enylene, non-4-enylene, dec-1-enylene, dec-2-enylene, hexa-1,3-dienylene, hexa-2,4-dienylene, hexa-1,3,5-trienylene, 1,3-cyclopent-4-enylene, 1,3-cylohex-4-enylene, and mixtures thereof.

Preferably, each R¹ in the oligoester ammonium salt of formula (I) is independently selected from compounds being linear or branched C₂₀-C₂₄-alkyl groups. More preferably, each R¹ is independently selected from compounds being linear or branched C₂₀-C₂₂-alkyl groups. In a preferred embodiment, R¹ is linear C₂₀-C₂₂-alkyl. In another preferred embodiment, R¹ is C₂₁-alkyl, which can be the alkyl part of behenic acid.

Preferably R² is a linear or branched C₁-C₈-alkylene or linear or branched C₂-C₈-alkenylene group. Preferably, -R²- in the oligoester ammonium salt is independently selected from the compounds being linear -(C₂-C₈-alkylene)-groups. Preferably,
- R²- in the oligoester ammonium salt is independently selected from the compounds being linear -(C₄-C₈-alkylene)- groups. More preferably, the compounds being "linear -(C₂-C₆-alkylene)-" groups are selected from the group consisting of
- CH2-CH2- (ethylene), -CH2-CH2-CH2- (propylene), -CH2-CH2-CH2-CH2-(butylene), -CH2-CH2-CH2-CH2-CH2- (pentylene),-CH2-CH2-CH2-CH2-CH2-CH2-(hexylene), and mixtures thereof. Particularly preferred for -R²- is
- CH2-CH2-CH2-CH2-, -CH2-CH2-CH2-CH2-CH2-CH2-CH2-CH2- or -CH2-CH2-CH2-CH2-CH2-CH2-. Most preferred for -R²- is -CH2-CH2-CH2-CH2-CH2-CH2-CH2-CH₂-.

In at least one embodiment of the present invention, in the oligoester ammonium salt -R³ is -(C₁-C₄-alkyl), preferably -R³ is methyl.

In the oligoester ammonium salt, R⁴ is preferably selected from hydrogen, methyl or ethyl. More preferably, R⁴ is methyl.

In at least one embodiment of the present invention, in the oligoester ammonium salt R^{x} is a linear or branched hydroxy-C₁-C₆-alkyl group, preferably linear or branched hydroxy-C₁-C₄-alkyl group, more preferably linear hydroxy-C₁-C₂-alkyl group, most preferably hydroxyethyl.

In at least one embodiment of the present invention, in the oligoester ammonium salt R^{y} is preferably selected from hydrogen, methyl or ethyl. More preferably, R^{y} is methyl.

In the context of the present invention, the letter "n" in general formula (I) defines the average number of repeating units of the oligoester ammonium salts. The number n is preferably an integer of from 1 to 30, more preferably from 1 to 10.

In the oligoester ammonium salt of general formula (I), the counter ion [(X^{p-})_{1/p}] of the (m) charged oligoester ammonium cation is preferably selected from the group consisting of: Cl⁻ (chloride), Br⁻ (bromide), MeSO₄⁻ (methyl-sulfate), EtSO₄⁻(ethyl sulfate), HCO₂⁻ (formate), citrate, acetate, nitrate, [(CO₃²⁻)_{0.5}] (carbonate), [(SO₄²⁻)_{0.5}] (sulfate), [(PO₄³⁻)_{1/3}] (phosphate), and combinations thereof.

The cosmetic composition comprises also one or more further components (F), which can be in an amount of at least 0.5% by weight, such as 0.5 to 20% by weight of the cosmetic composition. Preferably, the component (F) is selected from the group consisting of: acidity regulators, colorants, further conditioning agents, emulsifiers, film formers, fragrances, glossers, humectants, lubricants, moisturizers, pigments, preservatives, skin penetration enhancers, stabilizers, further surfactants, thickeners, and viscosity modifiers. More preferably, the component (F) is selected from the group consisting of acidity regulators, glossers, lubricants, and further surfactants.

Suitable lubricants are, for example, fatty alcohol components having 6 to 18 carbon atoms. The further surfactants may, for example, be chosen from non-polymeric, cationic quaternary ammonium compounds, in particular cetrimonium chloride (CTAC).

Suitable classical cationic conditioning agents include cationic quaternary ammonium salts. In at least one embodiment, the component (F) is a cationic quaternary ammonium salt. Examples of such quaternary ammonium salts include benzyl triethyl ammonium chloride, cetyl trimethylammonium chloride (cetrimonium chloride, CTAC), behentrimonium chloride (BTAC) and cetylpyridinium chloride.

As cationic components, a variety of further cationic polymers are suitable, including quaternized cellulose ethers, copolymers of vinylpyrrolidone, acrylic polymers, including homopolymers or copolymers of dimethyldiallylammonium chloride and acrylamide. Also suitable are various types of homo-or copolymers derived from acrylic or methacrylic acid, acrylamide, methylacrylamide, diacetoneacrylamide.

For the method of treating hair according to the invention, a shampoo composition can be used (which may or may not be according to the present invention), but the conditioner composition according to the present invention can also be used without using a shampoo composition.

In at least one embodiment, shampoo compositions comprise from 1 to 99%, preferably from 5 to 95%, more preferably from 10 to 90% by weight of the total composition of water, and from 0.1 to 99%, preferably from 1 to 95%, more preferably from 5 to 90%, often 5 to 25% by weight of the total composition of a cleansing surfactant. Suitable cleansing surfactants are generally anionic, amphoteric, betaine, or zwitterionic surfactants. Preferably, the anionic surfactants are alkyl ether or alkyl ether sulfates such as sodium lauryl sulfate, and other components described above.

In at least one embodiment, the component (F) is a glosser. Typical glossers are silicones. Suitable as silicones are volatile or nonvolatile nonionic silicone fluids, silicone resins, and silicone semisolids or solids. Volatile silicones are linear or cyclic silicones having a measureable vapor pressure, which is defined as a vapor pressure of at least 2 mm of mercury at 20°C. Also suitable are water insoluble nonvolatile silicone fluids including polyalkyl siloxanes, polyaryl siloxanes, polyalkylaryl siloxanes, polyether siloxane copolymers, amine-functional silicones, and mixtures thereof.

Typical oils to be used in hair compositions are organic oils, which often are esters. The organic oil component may also comprise glyceryl esters of fatty acids, or triglycerides, coconut oil, almond oil, apricot kernel oil, avocado oil, babassu oil, evening primrose oil, camelina sativa seed oil, grape seed oil, macadamia ternifolia seed oil, corn oil, meadowfoam seed oil, mink oil, olive oil, palm kernel oil, safflower oil, sesame oil, soybean oil, sunflower oil, wheat germ oil, and camellia reticulata seed oil. Also suitable as the oil component are sorbitan esters and glyceryl esters as described above. The cosmetic composition of the invention may contain from 0.05 to 5%, preferably 0.5 to 5% by weight of at least one oil component.

The composition of the invention can contain from 0.1 to 10% by weight, preferably from 0.2 to 5% by weight, more preferably from 0.5 to 5% by weight of at least one rheology modifying agent, in particular a gelling and thickening agent. Examples are cellulosic thickeners, for example, hydroxyethyl-cellulose, hydroxypropylcellulose, and carboxymethylcellulose, guar gum, such as hydroxypropylguar, gums of microbial origin, such as xanthan gum and scleroglucan gum, and synthetic thickeners, such as crosslinked homo- or copolymers of acrylic acid and/or of acrylamidopropanesulphonic acid. Other rheology modifying agents include fatty acid amides such as coconut diethanolamide and monoethanolamide, and oxyethylenated monoethanolamide of carboxylic acid alkyl ether.

Generally, the cosmetic composition according to the present invention is used for topical application to hair, but also skin or nail treatment is possible. In general, the cosmetic composition comprises 0.01 to 20% by weight of one or more oligoester ammonium salts as described above, in particular of formula (I), based on the total weight of the composition. More preferred, the oligoester ammonium salt is added in an amount from 0.05 to 20%, or from 0.1 to 15%, often 0.1 to 10% by weight based on the total weight of the composition.

In the case of hair conditioner compositions, the oligoester ammonium salt is added in an amount high enough to achieve at least one desired effect such as, for example, improved shine (gloss), detangling of hair or static charge reduction. Preferably, the hair conditioner composition comprises one or more oligoester ammonium salts of formula (I) in an amount of from 0.1 to 15% by weight based on the total weight of the composition. All percentages by weight given herein are based upon the total weight of the composition of the present invention, unless otherwise specified.

The hair conditioning composition of the present invention can also comprise as component (F) a fatty compound. The fatty compound is included in the composition at a level of from 0.1 to 20 % by weight, preferably from 1.0 to 10 % by weight. The fatty compound is selected from the group consisting of fatty alcohols (e.g. cetyl alkohol, stearyl alcohol or cetearyl alcohol), fatty acids, fatty alcohol derivatives, fatty acid derivatives, and mixtures thereof.

It is understood that the components disclosed can in some instances fall into more than one classification, e.g., some fatty alcohol derivatives can also be classified as fatty acid derivatives. However, a given classification, is not intended to be a limitation on that particular compound, but is done so for convenience of classification and nomenclature. Non-limiting examples are found in International Cosmetic Ingredient Dictionary and Handbook, Fourteenth Edition (2014), and CTFA Cosmetic Ingredient Handbook, Second Edition, 1992. Preferably, fatty alcohols have 14 to 30 or 16 to 22 carbon atoms. These fatty alcohols are saturated and can be linear or branched. Examples of fatty alcohols are cetyl alcohol, stearyl alcohol, behenyl alcohol, and mixtures thereof. Preferred fatty acids have from 10 to 30 or from 12 to 22 carbon atoms. These fatty acids can be saturated and can be linear or branched. Also included herein are salts of these fatty acids. Examples of fatty acids are lauric acid, palmitic acid, stearic acid, behenic acid, sebacic acid, and mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, and mixtures thereof.

Examples of fatty alcohol derivatives and fatty acid derivatives include methyl stearyl ether; polyoxyethylene ethers of behenyl alcohol; ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, poly glyceryl stearate, glyceryl monostearate, glyceryl distearate, glyceryl tristearate, and mixtures thereof.

The hair conditioning compositions of the present invention may comprise, in addition to the oligoester ammonium salt and at least one further component (F), an aqueous carrier. The level and species of the aqueous carrier are selected according to the compatibility with other components and other desired characteristic of the composition. The carrier includes water and water solutions of lower alkyl alcohols and polyhydric alcohols. The lower alkyl alcohols useful herein are monohydric alcohols having 1 to 6 carbons, often ethanol and/or isopropanol. The useful polyhydric alcohols include propylene glycol, hexylene glycol, glycerin, and propane diol. Preferably, the aqueous carrier is substantially water. Deionized water is preferably used. Water from natural sources, including minerals can also be used, depending on the desired characteristic of the cosmetic composition. Generally, the cosmetic compositions of the present invention can comprise up to 80%, often even up to 95 % by weight of water.

Preferably, the compositions of the present invention contains as a further component a silicone compound. The composition can comprise up to 5% (e.g. 0.1 to 5%) by weight of a silicone compound. Suitable silicone compounds include polyalkyl or polyaryl siloxanes. The preferred silicone compounds are polydimethylsiloxane, polydiethylsiloxane, and polymethylphenylsiloxane, e.g. available from Wacker (Germany) or Dow Corning, such as Xiameter PMX DC 200. The silicone compounds may further be incorporated in the present composition in the form of an emulsion, wherein the emulsion is made by mechanical mixing with or without the aid of an additional surfactant selected from anionic surfactants, nonionic surfactants, cationic surfactants, and mixtures thereof.

The compositions of the present invention may also include as a further component (F), other components being suitable for rendering the composition more cosmetically or aesthetically acceptable or to provide them with additional usage benefits. Such other components can generally be used individually at levels of from 0.001 % to 5 % by weight. A wide variety of further components (F) can be formulated into the present composition. These include other conditioning agents, such as hydrolysed collagen, vitamin E, or panthenol, panthenyl ethyl ether, hydrolysed keratin, proteins, plant extracts, nutrients; and emollients such as PPG-3 myristyl ether, trimethyl pentanol hydroxyethyl ether; hair-fixative polymers such as amphoteric fixative polymers, cationic fixative polymers, anionic fixative polymers, nonionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents; hair oxidizing (bleaching) agents, such as hydrogen peroxide, perborate and persulfate salts; hair reducing agents such as the thioglycolates; perfumes; and sequestering agents, such as disodium ethylene-diamine tetraacetate; ultraviolet and infrared screening and absorbing agents such as octyl salicylate; and anti-dandruff agents such as zinc pyrithione and salicylic acid.

The hair conditioning composition of the present invention may contain as a further component (F) a polysorbate for adjusting rheology, for example, polysorbate-20, polysorbate-21, polysorbate-40, polysorbate-60, and mixtures thereof. The polysorbate can be contained in the composition in amounts up to 5% (e.g. 0.1 to 5%) by weight.

The hair conditioning composition can also contain as a further component (F) a polypropylene glycol. Polypropylene glycols are those having a weight average molecular weight of from 200 to 100000 g/mol. The polypropylene glycol useful herein may be either water-soluble, water- insoluble, or may have a limited solubility in water, depending upon the degree of polymerization and whether other moieties are attached thereto. The desired solubility of the polypropylene glycol in water will depend in large part upon the form (e.g., leave-on, or rinse-off form) of the hair care composition.

For example, in a rinse-off hair care composition, it is preferred that the polypropylene glycol herein has a solubility in water at 25°C of less than about 1 g/100 g water, more preferably a solubility in water of less than about 0.5 g/100 g water, and even more preferably a solubility in water of less than about 0.1 g/100 g water. The polypropylene glycol can be included in the hair conditioning composition of the present invention at a level of up to 10% by weight.

The hair conditioning composition can also contain, as a further component (F), low melting point oil selected from the group consisting of hydrocarbons having from 10 to 40 carbon atoms; unsaturated fatty alcohols having from 10 to 30 carbon atoms such as oleyl alcohol; unsaturated fatty acids having from about 10 to about 30 carbon atoms; fatty acid derivatives; fatty alcohol derivatives; ester oils such as pentaerythritol ester oils, trirnethylol ester oils, citrate ester oils, and glyceryl ester oils; poly [alpha]-olefin oils; and mixitures thereof. Preferred low melting point oils herein are selected from the group consisting of: ester oils such as pentaerythritol ester oils, trimethylol ester oils, citrate ester oils, and glyceryl ester oils; poly [alpha]-olefin oils; and mixtures thereof, Particularly useful pentaerythritol ester oils and trimethylol ester oils herein include pentaerythritol tetraisostearate, pentaerythritol tetraoleate, trimethylolpropane triisostearate, trimethylolpropane trioleate, and mixtures thereof. Particularly useful glyceryl ester include triisostearin, triolein and trilinolein.

The hair conditioning composition can also contain, as a further component (F), a cationic polymer. Cationic polymers useful herein are those having an number average molecular weight of at least about 5000, typically from 10000 to 10 million (g/mol). Suitable cationic polymers include, for example, copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. Other suitable spacer monomers include vinyl esters, vinyl alcohol, maleic anhydride, propylene glycol, and ethylene glycol. Other suitable cationic polymers useful herein include, for example, cationic celluloses, cationic starches, and cationic guar gums. Commercially available cationic guar polymers are e.g. Jaguar^{®} from Rhodia or Solvay.

The hair conditioning composition can be in the form of rinse-off products or leave-on products, can be opaque, and can be formulated in a wide variety of product forms, including creams, gels, emulsions, mousses and sprays. Preferably, the hair conditioner composition is in the form of a rinse-off product.

In at least one embodiment, the cosmetic composition is a shampoo composition. In at least one embodiment, the shampoo composition comprises also one or more further cosmetically acceptable component (F), which can be present in an amount of at least 0.5% by weight, or from 0.5 to 20%, by total weight of the shampoo composition. Preferably, the component (F) is selected from the group consisting of: cleansing ingredients, acidity regulators, colorants, further conditioning agents, emulsifiers, film formers, fragrances, glossers, humectants, lubricants, moisturizers, pigments, preservatives, hair penetration enhancers, scalp actives, stabilizers, further surfactants, thickeners, viscosity modifiers, and combinations thereof. More preferably, the component (F) is selected from the group consisting of surfactants, viscosity-modifying polymers and conditioning ingredients. In at least one embodiment the shampoo composition comprises from 1 to 20% by weight of one or more oligoester ammonium salts (OAS) and further comprises at least 0.5% by weight of one or more further components (F) selected from the group consisting of surfactants, polymers, conditioning agents, actives, acidity regulators, lubricants, moisturisers, oils, preservatives, sequestrants, strengtheners, sun protectors, and combinations thereof.

In at least one embodiment, the shampoo composition comprises further cosmetically acceptable component (F) being cleansing ingredients. In at least one embodiment, the shampoo composition comprises from 0.05% to 20% cleansing ingredients. In at least one embodiment of the shampoo composition, the level of cleansing ingredient is from 1% to 20% by total weight of the active ingredient in the composition, preferably 5% to 18%, more preferably 8% to 16%. In at least one embodiment, the cleansing ingredient is selected from the group consisting of non-polymeric surfactants, saponins, polymeric surfactants, and combinations thereof. Preferably the cleansing ingredient comprises or consists of surfactants.

In at least one embodiment the shampoo composition preferably comprises from 0.01 to 20 % by weight of one or more OAS and at least 0.5 % by weight of further surfactant(s), preferably cleansing anionic or nonionic surfactants, such as sodium laureth sulphate, sodium lauryl sulphate, ammonium laureth sulphate, ammonium lauryl sulphate, olefin sulfonates, olefin sulfates, laureth-3 or 4, cocamide DEA, glucosides, cocamidopropyl betaine, coco betaine, cocoamphodipropionate, sodium methyl 2-sulfolaurate and other laurates, sulfoacetates, sulfosuccinates, lactylates, sultaines, caprylates/ caprates, isethionates, glutamates, taurates, sarcosinates, glucamides, and combinations thereof.

In at least one embodiment, the composition comprises a surfactant system. In at least one embodiment, the surfactant system comprises a surfactant selected from the group consisting of anionic surfactants, cationic surfactants, non-ionic surfactants, zwitterionic surfactants and/or amphoteric surfactants.

In at least one embodiment, the composition comprises a total amount of surfactant of from 0.01 wt.-% to 70 wt.-%, from 0.1 wt.-% to 40%, from 1 wt.-% to 30%, from 2 wt.-% to 20 wt.-%.

In at least one embodiment, the composition comprises an anionic surfactant. In at least one embodiment, the anionic surfactant is selected from the group consisting of (C₁₀-C₂₀)-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkanesulfonates and hydroxyalkanesulfonates, olefinsulfonates, acyl esters of isethionates, α-sulfo fatty acid esters, alkylbenzenesulfonates, alkylphenol glycol ether sulfonates, sulfosuccinates, sulfosuccinic monoesters and diesters, fatty alcohol ether phosphates, protein/fatty acid condensation products, alkyl monoglyceride sulfates and sulfonates, alkylglyceride ether sulfonates, fatty acid methyltaurides, fatty acid sarcosinates, sulforicinoleates, acylglutamates, and mixtures thereof. The anionic surfactants (and their mixtures) can be used in the form of their water-soluble or water-dispersible salts, examples being the sodium, potassium, magnesium, ammonium, mono-, di-, and triethanolammonium, and analogous alkylammonium salts. In at least one embodiment, the anionic surfactant is the salt of an anionic surfactant comprising 12 to 14 carbon atoms. In at least one embodiment, the anionic surfactant is selected from the group consisting of sodium lauryl sulfate, sodium laureth sulfate, sodium tridecyl sulfate, sodium trideceth sulfate, sodium myristyl sulfate, sodium myreth sulfate, and mixtures thereof. Typical anionic surfactants for use in compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecyl benzene sulphonate, triethanolamine dodecylbenzene sulphonate, sodium cocoyl isethionate, sodium lauryl isethionate, lauryl ether carboxylic acid and sodium N-lauryl sarcosinate. Preferred anionic surfactants are selected from sodium lauryl sulphate and sodium lauryl ether sulphate(n)EO, (where n is from 1 to 3); more preferably sodium lauryl ether sulphate(n)EO, (where n is from 1 to 3); most preferably sodium lauryl ether sulphate(n)EO where n=1 . Preferably the level of alkyl ether sulphate is from 0.5 wt.-% to 25 wt.-% of the total composition, more preferably from 3 wt.-% to 18 wt.-%, most preferably from 6 wt.-% to 15 wt.-% of the total composition.

The total amount of anionic surfactant in compositions of the invention may range from 0.5 wt.-% to 45 wt.-%, more preferably from 1.5 wt.-% to 20 wt.-%. Compositions of the invention may comprise fatty acyl isethionate, if present preferably at a level of from 1 to 10 wt.-%, more preferably from 2 to 8 wt.-%, most preferably from 2.5 to 7.5 wt.-%. A preferred fatty acyl isethionate product comprises fatty acyl isethionate surfactant at a level of from 40 to 80 wt.-% of the product, as well as free fatty acid and/or fatty acid salt at a level of from 15 to 50 %.

Preferably, greater than 20 wt.-% and less than 45 wt.-%, more preferably greater than 25 wt.-% and less than 45 wt.-% of the fatty acyl isethionate are of chain length greater than or equal to C16; and greater than 50 wt.-%, preferably greater than 60 wt.-% of the free fatty acid/soap is of chain length C16 to C20.

In addition, the product may contain isethionates salts which are present typically at levels less than 5 wt.-%, and traces (less than 2 wt.-%) of other impurities.

Preferably, a mixture of aliphatic fatty acids is used for the preparation of commercial fatty acyl isethionates surfactants. The resulting fatty acyl isethionate surfactants (e.g., resulting from reaction of alkali metal isethionate and aliphatic fatty acid) preferably should have more than 20 wt.-%, preferably more than 25 wt.-%, but no more than 45 wt.-%, preferably 35 % (on basis of fatty acyl isethionates reaction product) of fatty acyl group with 16 or greater carbon atoms to provide both excellent lather and mildness of the resulting fatty acyl isethionate product. These longer chain fatty acyl isethionate surfactants and fatty acids, i.e. fatty acyl group and fatty acid with 16 or more carbons, can typically form insoluble surfactant/fatty acid crystals in water at ambient temperatures.

In at least one embodiment, the composition comprises an acylglycinate surfactant. In at least one embodiment, the acylglycinate surfactant conforms to the formula (Y): 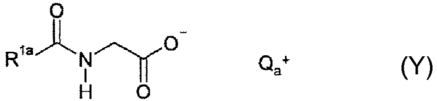 wherein
R^{1a} is a linear or branched, saturated alkanoyl group having 6 to 30, preferably 8 to 22, particularly preferably 8 to 18, carbon atoms or is a linear or branched, mono- or polyunsaturated alkenoyl group having 6 to 30, preferably 8 to 22 and particularly preferably 12 to 18 carbon atoms, and
Qa⁺ is a cation.

In at least one embodiment, Qa⁺ is selected from the group consisting of Li⁺, Na⁺, K⁺, Mg⁺⁺, Ca⁺⁺, Al⁺⁺⁺, NH₄⁺, a monoalkylammmonium ion, a dialkylammonium ion, a trialkylammonium ion and a tetraalkylammonium ion, or combinations thereof. Optionally R^{1a} is independently from one another, are (C₁-C₂₂)-alkyl radicals or (C₂-C₁₀)-hydroxyalkyl radicals. In at least one embodiment, the acylglycinate surfactant is selected from sodium cocoylglycinate and potassium cocoylglycinate. In at least one embodiment, the acylglycinate surfactant is selected from those conforming to formula (Y), wherein R is C12 alkyl or C14 alkyl. In at least one embodiment, the acylglycinate surfactant is selected from those conforming to formula (Y), wherein R is C16 alkyl or C18 alkyl.

In at least one embodiment, the composition comprises from 0.01 wt.-% to 30 wt.-%, or 1 wt.-% to 25 wt.-%, preferably from 5 wt.-% to 20 wt.-%, more preferably from 12 wt.-% to 18 wt.-% anionic surfactant.

In at least one embodiment, the composition comprises a glutamate surfactant corresponding to formula (Z) or a salt thereof: 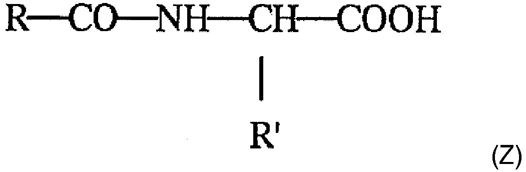 wherein R' is HOOC-CH₂-CH₂- or M⁺⁻OOC-CH₂-CH₂- wherein M⁺ is a cation; and wherein R is a linear or branched, saturated alkanoyl group having 6 to 30, preferably 8 to 22, more preferably 8 to 18, carbon atoms or is a linear or branched, mono- or polyunsaturated alkenoyl group having 6 to 30, preferably 8 to 22 and more preferably 12 to 18 carbon atoms. In at least one embodiment, M⁺ is a metal cation. In at least one embodiment, M⁺ is selected from the group consisting of Li⁺, Na⁺, K⁺, Mg⁺⁺, Ca⁺⁺, Al⁺⁺⁺, NH₄⁺, a monoalkylammmonium ion, a dialkylammonium ion, a trialkylammonium ion and a tetraalkylammonium ion, or combinations thereof. In at least one embodiment, the glutamate surfactant is selected from sodium cocoyl glutamate and potassium cocoyl glutamate. In at least one embodiment, the glutamate surfactant is selected from those conforming to formula (Z), wherein R is C12 alkyl or C14 alkyl. In at least one embodiment, the glutamate surfactant is selected from those conforming to formula (Z), wherein R is C16 alkyl or C18 alkyl.

In at least one embodiment, the composition comprises a non-ionic surfactant. The non-ionic surfactants may bepresent in the range 0 to 5 wt.-%. Nonionic surfactants that can be included in compositions of the invention include condensation products of aliphatic primary or secondary linear or branched chain alcohols or phenols with alkylene oxides, usually ethylene oxide and generally having from 6 to 30 ethylene oxide groups. Alkyl ethoxylates are particularly preferred. Most preferred are alky ethoxylates having the formula

R-(OCH2CH2)nOH,

where R is an alkyl chain of C12 to C15, and n is 5 to 9. Other suitable nonionic surfactants include mono- or di-alkyl alkanolamides. Examples include coco mono- or di-ethanolamide and coco mono-isopropanolamide.

Further nonionic surfactants which can be included in compositions of the invention are the alkyl polyglycosides (APGs). Typically, APG is one which comprises an alkyl group connected (optionally via a bridging group) to a block of one or more glycosyl groups. Preferred APGs are defined by the following formula:

RO-(G)n

wherein R is a branched or straight chain alkyl group which may be saturated or unsaturated and G is a saccharide group. R may represent a mean alkyl chain length of from about C5 to about C20. Preferably R represents a mean alkyl chain length of from about C9 to about C12. G may be selected from C5 or C6 monosaccharide residues, and is preferably a glucoside. G may be selected from the group comprising glucose, xylose, lactose, fructose, mannose and derivatives thereof. Preferably G is glucose. The degree of polymerisation, n, may have a value of from about 1 to about 10 or more. Most preferably the value of n lies from about 1 .3 to about 1 .5. Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the fatty (e.g. C10-C18) N-alkyl (C1-C6) polyhydroxy fatty acid amides, such as the C12-C18 N-methyl glucamides, as described for example in WO9206154 and US5194639, and the N-alkoxy polyhydroxy fatty acid amides.

In at least one embodiment, the non-ionic surfactant has an HLB (Hydrophilic Lipophilic Balance) of greater than 12. Optionally, the non-ionic surfactant is selected from the group consisting of ethoxylated or ethoxylated/propoxylated fatty alcohols with a fatty chain comprising from 12 to 22 carbon atoms, ethoxylated sterols, such as stearyl- or lauryl alcohol (EO-7), PEG-16 soya sterol or PEG-10 soya sterol, polyoxyethylene polyoxypropylene block polymers (poloxamers), and mixtures thereof.

In at least one embodiment, the non-ionic surfactant is selected from the group consisting of ethoxylated fatty alcohols, fatty acids, fatty acid glycerides or alkylphenols, in particular addition products of from 2 to 30 mol of ethylene oxide and/or 1 to 5 mol of propylene oxide onto C8- to C22-fatty alcohols, onto C12- to C22-fatty acids or onto alkyl phenols having 8 to 15 carbon atoms in the alkyl group, C12- to C22-fatty acid mono- and diesters of addition products of from 1 to 30 mol of ethylene oxide onto glycerol, addition products of from 5 to 60 mol of ethylene oxide onto castor oil or onto hydrogenated castor oil, fatty acid sugar esters, in particular esters of sucrose and one or two C8- to C22-fatty acids, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate, esters of sorbitan and one, two or three C8- to C22-fatty acids and a degree of ethoxylation of from 4 to 20, polyglyceryl fatty acid esters, in particular of one, two or more C8- to C22-fatty acids and polyglycerol having preferably 2 to 20 glyceryl units, alkyl glucosides, alkyl oligoglucosides and alkyl polyglucosides having C8 to C22-alkyl groups, e.g. decylglucoside or laurylglucoside, and mixtures thereof.

In at least one embodiment, the non-ionic surfactant is selected from the group consisting of fatty alcohol ethoxylates (alkylpolyethylene glycols), alkylphenol polyethylene glycols, alkylmercaptan polyethylene glycols, fatty amine ethoxylates (alkylaminopolyethylene glycols), fatty acid ethoxylates (acylpolyethylene glycols), polypropylene glycol ethoxylates (Pluronics^{®}), fatty acid alkylol amides, (fatty acid amide polyethylene glycols), N-alkyl-, N-alkoxypolyhydroxy-fatty acid amide, sucrose esters, sorbitol esters, polyglycol ethers, and mixtures thereof.

In at least one embodiment, the composition comprises a fatty N-methyl-N-glucamide surfactant. In at least one embodiment, the fatty N-methyl-N-glucamide surfactant conforms to the formula (X): 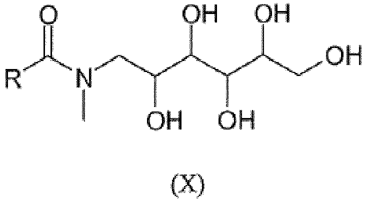 wherein
R is a a linear or branched alkyl or alkenyl group having from 3 to 30 carbon atoms. In at least one embodiment, R is an alkyl group having from 3 to 30 carbon atoms. In at least one embodiment, R is a saturated aliphatic hydrocarbon group which can be linear or branched and can have from 3 to 20 carbon atoms in the hydrocarbon chain, preferably linear or branched. Branched means that a lower alkyl group such as methyl, ethyl or propyl is present as substituent on a linear alkyl chain. In at least one embodiment, R is selected from the group consisting of 1-propyl, 2-propyl, 1-butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, 1-heptyl, 1-octyl, 1-nonyl, 1-decyl, 1-undecyl, 1-dodecyl, 1-tetradecyl, 1-hexadecyl and 1-octadecyl. Suitable fatty N-methyl-N-glucamide surfactants are described in WO2013/178700 and
EP 0 550 637, which are incorporated herein by reference. In at least one embodiment, the N-methyl-N-glucamide surfactant is selected from those conforming to formula (X), wherein R is C12 alkyl or C14 alkyl. In at least one embodiment, the N-methyl-N-glucamide surfactant is selected from those conforming to formula (X), wherein R is C16 alkyl or C18 alkyl.

In at least one embodiment, the composition comprises from 1 wt.-% to 20 wt.-%, more preferably from 2 wt.-% to 10 wt.-%, even more preferably from 3 wt.-% to 7 wt.-% non-ionic surfactant.

Amphoteric or zwitterionic surfactant can be included in the composition in an amount ranging from 0.5 wt.-% to about 8 wt.-%, preferably from 1 wt.-% to 4 wt.-% of the total composition.

In at least one embodiment, the amphoteric surfactants are selected from the group consisting of N-(C₁₂-C₁₈)-alkyl-β-aminopropionates and N-(C₁₂-C₁₈)-alkyl-β-iminodipropionates as alkali metal salts and mono-, di-, and trialkylammonium salts; N-acylaminoalkyl-N,N-dimethylacetobetaine, preferably N-(C₈-C₁₈)-acylaminopropyl-N,N-dimethylacetobetaine, (C₁₂-C₁₈)-alkyl-dimethyl-sulfopropylbetaine, amphosurfactants based on imidazoline (trade name: Miranol^{®}, Steinapon^{®}), preferably the sodium salt of 1-(β-carboxymethyloxyethyl)-1-(carboxymethyl)-2-laurylimidazolinium; amine oxide, e.g., (C₁₂-C₁₈)-alkyl-dimethylamine oxide, fatty acid amidoalkyldimethylamine oxide, and mixtures thereof.

In at least one embodiment, the composition comprises a betaine surfactant. Optionally, the betaine surfactant is selected from C8- to C18-alkylbetaines. In at least one embodiment, the betaine surfactant is selected from the group consisting of cocodimethylcarboxymethylbetaine, lauryldimethylcarboxymethylbetaine, lauryldimethylalphacarboxyethylbetaine, cetyldimethylcarboxymethylbetaine, oleyldimethylgammacarboxypropylbetaine and laurylbis(2-hydroxypropyl)alphacarboxyethylbetaine and combinations thereof. Optionally, the betaine surfactant is selected from C8- to C18-sulfobetaines. In at least one embodiment, the betaine surfactant is selected from the group consisting of cocodimethylsulfopropylbetaine, stearyldimethylsulfopropylbetaine, lauryldimethylsulfoethylbetaine, laurylbis(2-hydroxyethyl)sulfopropylbetaine, and combinations thereof. Optionally, the betaine surfactant is selected from carboxyl derivatives of imidazole, the C8- to C18-alkyldimethylammonium acetates, the C8- to C18-alkyldimethylcarbonylmethylammonium salts, and the C8- to C18-fatty acid alkylamidobetaines, and mixtures thereof. Optionally, the C8- to C18-fatty acid alkylamidobetaine is selected from coconut fatty acid amidopropylbetaine, N-coconut fatty acid amidoethyl-N-[2-(carboxymethoxy)ethyl]glycerol (CTFA name: Cocoamphocarboxyglycinate), and mixtures thereof. A particularly preferred amphoteric or zwitterionic surfactant is cocamidopropyl betaine. Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

In at least one embodiment, the composition comprises from 0.5 wt.-% to 20 wt.-%, preferably from 1 wt.-% to 10 wt.-% amphoteric surfactant.

In at least one embodiment, the composition comprises a surfactant system. In at least one embodiment, the surfactant system comprises at least one surfactant selected from the group consisting of lauryl sulfate, laureth sulfate, cocoamidopropylbetaine, sodium cocoylglutamate, lauroamphoacetate, and mixtures thereof. In at least one embodiment, the surfactant system comprises sodium laureth sulphate, sodium lauryl sulphate, and optionally cocamidopropyl betaine. In at least one embodiment, the surfactant system comprises sodium laureth sulphate, Potassium Cocyl Glutamate, and cocamidopropyl betaine.

Suitable lubricants are, for example, fatty alcohol components having 6 to 18 carbon atoms. The further surfactants may, for example, be chosen from non-polymeric, cationic quaternary ammonium compounds, in particular cetrimonium chloride (CTAC).

Suitable classical cationic conditioning agents include cationic quaternary ammonium salts. Examples of such quaternary ammonium salts include benzyl triethyl ammonium chloride, cetyl trimethylammonium chloride (cetrimonium chloride, CTAC), behentrimonium chloride (BTAC) and cetylpyridinium chloride.

As cationic components, a variety of further cationic polymers are suitable, including quaternized cellulose ethers, copolymers of vinylpyrrolidone, acrylic polymers, including homopolymers or copolymers of dimethyldiallylammonium chloride and acrylamide. Also suitable are various types of homo-or copolymers derived from acrylic or methacrylic acid, acrylamide, methylacrylamide, diacetoneacrylamide.

Typical glossers are silicones. Suitable as silicones are volatile or nonvolatile nonionic silicone fluids, silicone resins, and silicone semisolids or solids. Volatile silicones are linear or cyclic silicones having a measureable vapor pressure, which is defined as a vapor pressure of at least 2 mm of mercury at 20°C. Also suitable are water insoluble nonvolatile silicone fluids including polyalkyl siloxanes, polyaryl siloxanes, polyalkylaryl siloxanes, polyether siloxane copolymers, amine-functional silicones, and mixtures thereof.

Preferably, the compositions of the present invention contains as a further component a silicone compound. The composition can comprise up to 5% (e.g. 0.1 to 5%) by weight of a silicone compound. Suitable silicone compounds include polyalkyl or polyaryl siloxanes. The preferred silicone compounds are polydimethylsiloxane, polydiethylsiloxane, and polymethylphenylsiloxane, e.g. available from Wacker (Germany) or Dow Corning, such as Xiameter PMX DC 200. Silicone compounds can be available as silicone oils or emulsions. The silicone compounds may further be incorporated in the present composition in the form of an emulsion, wherein the emulsion is pre-made and added to the formulation, or made during the formulation process by mechanical mixing with or without the aid of an additional surfactant selected from anionic surfactants, nonionic surfactants, cationic surfactants, and mixtures thereof.

The compositions of the invention contain silicone conditioning agents preferably these are emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31 188. The viscosity of the emulsified silicone itself (not the emulsion or the final hair conditioning composition) is typically at least 10,000 cSt at 25°C the viscosity of the silicone itself is preferably at least 60,000 cSt, most preferably at least 500,000 cSt, ideally at least 1,000,000 cSt. Preferably the viscosity does not exceed 1×10⁹ cSt for ease of formulation. Emulsified silicones for use in the shampoo compositions of the invention will typically have an average silicone droplet size in the composition of less than 30, preferably less than 20, more preferably less than 10 micron, ideally from 0.01 to 1 micron. Silicone emulsions having an average silicone droplet size of less than 0.15 micron are generally termed microemulsions.

Silicone particle size may be measured by means of a laser light scattering technique, for example using a 2600D Particle Sizer from Malvern Instruments. Examples of suitable pre-formed emulsions include Xiameter MEM 1785 and microemulsion DC2-1865 available from Dow Corning. These are emulsions / microemulsions of dimethiconol. Cross-linked silicone gums are also available in a pre-emulsified form, which is advantageous for ease of formulation. A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non-ionic and/or cationic surfactant. Pre-formed emulsions of amino functional silicone are also available from suppliers of silicone oils such as Dow Corning and General Electric. Specific examples include DC939 Cationic Emulsion and the non-ionic emulsions DC2-7224, DC2- 8467, DC2-8177 and DC2-8154 (all ex Dow Corning).

Combination of amino and non amino functional silicones may also be used.

The total amount of silicone is preferably from 0.01 wt.-% to 10 wt.-% of the total composition more preferably from 0.1 wt.-% to 5 wt.-%, most preferably 0.5 wt.-% to 3 wt.-%.

Typical oils to be used in hair compositions are organic oils, which often are esters. The organic oil component may also comprise glyceryl esters of fatty acids, or triglycerides, coconut oil, almond oil, apricot kernel oil, avocado oil, babassu oil, evening primrose oil, camelina sativa seed oil, grape seed oil, macadamia ternifolia seed oil, corn oil, meadowfoam seed oil, mink oil, olive oil, palm kernel oil, safflower oil, sesame oil, soybean oil, sunflower oil, wheat germ oil, and camellia reticulata seed oil. Also suitable as the oil component are sorbitan esters and glyceryl esters as described above. The cosmetic composition of the invention may contain from 0.05 to 5%, preferably 0.5 to 5% by weight of at least one oil component.

The composition of the invention can contain from 0.1 to 10% by weight, preferably from 0.2 to 5% by weight, more preferably from 0.2 to 3% by weight of at least one rheology modifying agent, in particular a gelling and thickening agent. Examples are cellulosic thickeners, for example, hydroxyethyl-cellulose, hydroxypropylcellulose, and carboxymethylcellulose, guar gum, such as hydroxypropylguar, gums of microbial origin, such as xanthan gum and scleroglucan gum, and synthetic thickeners, such as crosslinked homo- or copolymers of acrylic acid and/or of acrylamidopropanesulphonic acid. Other rheology modifying agents include fatty acid amides such as coconut diethanolamide and monoethanolamide, and oxyethylenated monoethanolamide of carboxylic acid alkyl ether.

Rheology modifying agents are also known as structuring materials. Common structuring materials include polymeric materials known as "carbomers", including, for example the cross-linked polyacrylic acid polymers available from Lubrizol Corporation under the trademark Carbopol^{®}. Another class of (meth)acylic acid polymers are alkali-swellable emulsion (ASE) polymers. ASE polymers include, for example, Aculyn^{®} 38 copolymer from Dow. Carbomers and ASE polymers belong to a class of materials known as hydrodynamic thickeners. These hydrodynamic thickeners include acid groups in their polymeric structure that, when deprotonated, form anionic charges that repel each other, causing the polymer chains to expand and entangle. Expansion and chain entanglement can give rise to thickening and suspending effects provided by the deprotonated polymers. The properties of these hydrodynamic thickeners are impacted by their molecular weight, acid group content, degree of cross-linking, and extent of swelling. These thickeners are also known as "space filling" or "volume excluding", and tend to increase both viscosity and yield point as the concentration thereof is increased. In use, hydrodyamic polymers commonly give rise to compositions that exhibit shear thinning or non-Newtonian behavior. Another class of (meth)acrylic acid based rheology modifiers are hydrophobically modified alkali swellable (HASE) polymers.

Like ASE polymers, the HASE polymers include acid groups, the deprotonation of which gives rise to polymer swelling. Additionally, the HASE polymers include hydrophobic side groups, chains or blocks that give rise to associative interactions with each other, as well as with other hydrophobic species present in the compositions in which they are employed, for example, hydrophobic groups of surfactants, fatty acids, other thickening agents, and the like. Association creates hydrophobic regions distributed throughout the polymer chain network. This can also help to enhance the properties of the materials as solubilizing agents. Aculyn^{®} 22 and Aculyn^{®} 28 copolymers from Dow and Aqua SF-1^{®} copolymer from Lubrizol Corporation are among the commonly used HASE materials. U.S. Patent 4,529,773 (Witiak et al.) reports alkali-soluble emulsion polymers activated by neutralization to a pH above 6.5, and subsequently acidified in the presence of a surfactant. These are described as useful thickeners in acidic compositions. The polymers are formed from the copolymerization of a monomer system that includes: (1 ) methacrylic or acrylic acid, (2) methacrylic or acrylic acid ester of a C8-C30 alkyl or, as therein more particularly described, a hydrocarbyl monoether of polyethylene glycol, (3) a Ci- C4 alkyl acrylate or methacrylate, and, optionally, (4) a small amount of a polyethylenically unsaturated monomer.

Generally, the shampoo composition according to the present invention is used for topical application to hair, but also skin or nail treatment is possible. The composition is also suitable for cleansing animal hair, preferably mammalian hair. In general, the shampoo composition comprises 0.01 to 20% by weight of one or more oligoester ammonium salts as described above, in particular of formula (I), based on the total weight of the composition. More preferred, the oligoester ammonium salt is added in an amount from 0.05 to 20%, or from 0.1 to 15%, often 0.1 to 10% by weight based on the total weight of the composition.

In the shampoo compositions, the oligoester ammonium salt is added in an amount high enough to achieve at least one desired effect such as, for example, improved shine (gloss), detangling of hair or static charge reduction. Preferably, the shampoo composition comprises one or more oligoester ammonium salts of formula (I) in an amount of from 0.1 to 15% by weight based on the total weight of the composition. All percentages by weight given herein are based upon the total weight of the composition of the present invention, unless otherwise specified.

The shampoo composition of the present invention can also comprise as component (F) a fatty compound. The fatty compound is included in the composition at a level of from 0.1 to 20% by weight, preferably from 1.0 to10% by weight. The fatty compound is selected from the group consisting of fatty alcohols (e.g. cetyl alkohol, stearyl alcohol or cetearyl alcohol), fatty acids, fatty alcohol derivatives, fatty acid derivatives, and mixtures thereof.

It is understood that the components disclosed can in some instances fall into more than one classification, e.g., some fatty alcohol derivatives can also be classified as fatty acid derivatives. However, a given classification, is not intended to be a limitation on that particular compound, but is done so for convenience of classification and nomenclature. Non-limiting examples are found in International Cosmetic Ingredient Dictionary and Handbook, Fourteenth Edition (2014), and CTFA Cosmetic Ingredient Handbook, Second Edition, 1992. Preferably, fatty alcohols have 14 to 30 or 16 to 22 carbon atoms. These fatty alcohols are saturated and can be linear or branched. Examples of fatty alcohols are cetyl alcohol, stearyl alcohol, behenyl alcohol, and mixtures thereof. Preferred fatty acids have from 10 to 30 or from 12 to 22 carbon atoms. These fatty acids can be saturated and can be linear or branched. Also included herein are salts of these fatty acids. Examples of fatty acids are lauric acid, palmitic acid, stearic acid, behenic acid, sebacic acid, and mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, and mixtures thereof. Examples of fatty alcohol derivatives and fatty acid derivatives include methyl stearyl ether; polyoxyethylene ethers of behenyl alcohol; ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, poly glyceryl stearate, glyceryl monostearate, glyceryl distearate, glyceryl tristearate, and mixtures thereof.

Compositions according to the present invention may also comprise a dispersed, non-volatile, water-insoluble oily conditioning agent. By "insoluble" is meant that the material is not soluble in water (distilled or equivalent) at a concentration of 0.1 % (w/w), at 25°C.

Suitable oily or fatty materials are selected from hydrocarbon oils, fatty esters and mixtures thereof. Straight chain hydrocarbon oils will preferably contain from about 12 to about 30 carbon atoms. Also suitable are polymeric hydrocarbons of alkenyl monomers, such as C2-C6 alkenyl monomers. Specific examples of suitable hydrocarbon oils include: paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols, Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used.

Particularly preferred fatty esters are mono-, di- and triglycerides, more specifically the mono-, di-, and tri-esters of glycerol and long chain carboxylic acids such as Ci- C22 carboxylic acids. Preferred materials include cocoa butter, palm stearin, sunflower oil, soyabean oil and coconut oil.

The oily or fatty material is suitably present at a level of from 0.05 to 10, preferably from 0.2 to 5, more preferably from about 0.5 to 3 wt.-%.

The shampoo compositions of the present invention may comprise, in addition to the oligoester ammonium salt and at least one further component (F), an aqueous carrier. The level and species of the aqueous carrier are selected according to the compatibility with other components and other desired characteristic of the composition. The carrier includes water and water solutions of lower alkyl alcohols and polyhydric alcohols. The lower alkyl alcohols useful herein are monohydric alcohols having 1 to 6 carbons, often ethanol and/or isopropanol. The useful polyhydric alcohols include propylene glycol, hexylene glycol, glycerin, and propane diol. Preferably, the aqueous carrier is substantially water. Deionized water is preferably used. Water from natural sources, including minerals can also be used, depending on the desired characteristic of the cosmetic composition. Generally, the cosmetic compositions of the present invention can comprise up to 80 %, often even up to 95 % by weight of water.

The shampoo compositions of the present invention may also include as a further component (F), other components being suitable for rendering the composition more cosmetically or aesthetically acceptable or to provide them with additional usage benefits. Such other components can generally be used individually at levels of from 0.001 % to 5% by weight. A wide variety of further components (F) can be formulated into the present composition. These include other conditioning agents, such as hydrolysed collagen, vitamin E, or panthenol, panthenyl ethyl ether, hydrolysed keratin, proteins, plant extracts, nutrients; and emollients such as PPG-3 myristyl ether, trimethyl pentanol hydroxyethyl ether; hair-fixative polymers such as amphoteric fixative polymers, cationic fixative polymers, anionic fixative polymers, nonionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents; hair oxidizing (bleaching) agents, such as hydrogen peroxide, perborate and persulfate salts; hair reducing agents such as the thioglycolates; perfumes; and sequestering agents, such as disodium ethylene-diamine tetraacetate; ultraviolet and infrared screening and absorbing agents such as octyl salicylate; and anti-dandruff agents such as zinc pyrithione and salicylic acid.

In at least one embodiment, the composition comprises an anti-fungal substance. In at least one embodiment, the anti-fungal substance is selected from the group consisting of ketoconazole, oxiconazole, bifonazole, butoconazole, cloconazole, clotrimazole, econazole, enilconazole, fenticonazole, isoconazole, miconazole, sulconazole, tioconazole, fluconazole, itraconazole, terconazole, naftifine and terbinafine, zinc pyrithione, octopirox, and combinations thereof. In at least one embodiment, the composition comprises a total amount of anti-fungal substance in the composition of from 0.1 wt.-% to 1 wt.-%. In at least one embodiment, the composition comprises a pyridinethione anti-dandruff particulates, for example 1-hydroxy-2-pyridinethione salts, are highly preferred particulate anti-dandruff agents. The concentration of pyridinethione antidandruff particulate may ranges from 0.1% to 4%, by weight of the formulation, preferably from 0.1% to 3%, more preferably from 0.3% to 2%. Preferred pyridinethione salts include those formed from heavy metals such as zinc, tin, cadmium, magnesium, aluminum and zirconium, preferably zinc, more preferably the zinc salt of 1-hydroxy-2-pyridinethione (known as "zinc pyridinethione" or "ZPT"), more preferably 1-hydroxy-2-pyridinethione salts in platelet particle form. Salts formed from other cations, such as sodium, may also be suitable. Pyridinethione anti-dandruff agents are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080;

U.S. Pat. No. 4,323,683; U.S. Pat. No. 4,379,753; and U.S. Pat. No. 4,470,982. It is contemplated that when ZPT is used as the anti-dandruff particulate in the compositions herein, that the growth or regrowth of hair may be stimulated or regulated, or both, or that hair loss may be reduced or inhibited, or that hair may appear thicker or fuller.

Preferably salt is present at levels from 0.1 to 1 wt.-% of the total composition to adjust the product viscosity. Preferably NaOH is present at levels from 0.1 to 1 wt.-% of the total composition to adjust the pH of the formulation.

The shampoo composition of the present invention may contain as a further component (F) a polysorbate for adjusting rheology, for example, polysorbate-20, polysorbate-21, polysorbate-40, polysorbate-60, and mixtures thereof. The polysorbate can be contained in the composition in amounts up to 5% (e.g. 0.1 to 5%) by weight.

The shampoo composition can also contain as a further component (F) a polypropylene glycol. Polypropylene glycols are those having a weight average molecular weight of from 200 to 100000 g/mol. The polypropylene glycol useful herein may be either water-soluble, water-insoluble, or may have a limited solubility in water, depending upon the degree of polymerization and whether other moieties are attached thereto. The desired solubility of the polypropylene glycol in water will depend in large part upon the form of the shampoo composition. The polypropylene glycol can be included in the shampoo composition of the present invention at a level of up to 10% by weight.

The shampoo composition can also contain, as a further component (F), low melting point oil selected from the group consisting of hydrocarbons having from 10 to 40 carbon atoms; unsaturated fatty alcohols having from 10 to 30 carbon atoms such as oleyl alcohol; unsaturated fatty acids having from about 10 to about 30 carbon atoms; fatty acid derivatives; fatty alcohol derivatives; ester oils such as pentaerythritol ester oils, trirnethylol ester oils, citrate ester oils, and glyceryl ester oils; poly [alpha]-olefin oils; and mixitures thereof. Preferred low melting point oils herein are selected from the group consisting of: ester oils such as pentaerythritol ester oils, trimethylol ester oils, citrate ester oils, and glyceryl ester oils; poly [alpha]-olefin oils; and mixtures thereof. Particularly useful pentaerythritol ester oils and trimethylol ester oils herein include pentaerythritol tetraisostearate, pentaerythritol tetraoleate, trimethylolpropane triisostearate, trimethylolpropane trioleate, and mixtures thereof. Particularly useful glyceryl ester include triisostearin, triolein and trilinolein.

The shampoo composition can also contain, as a further component (F), a cationic polymer. Cationic polymers may be present in the composition of the invention for further enhancing deposition performance.

Suitable cationic polymers may be homopolymers which are cationically substituted or may be formed from two or more types of monomers. The weight average (M_{w}) molecular weight of the polymers will generally be between 100 000 and 2 million g/mol. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain non-cationic spacer monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1 -C7 alkyl groups, more preferably C1 -3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for exampl cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively; mineral acid salts of aminoalkyl esters of homo-and co-polymers of unsaturated carboxylic acids having from 3 to 5 carbon atoms, (as described in US4009256A1 from NAT STARCH CHEM CORP); cationic polyacrylamides (as described in WO95/22311A1 Unilever PLC).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula: A-O-[R-N⁺(R¹)(R²)(R³)X"], wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyi, alkoxyalkyi, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the trade name Polymer LM-200. Other suitable cationic polysaccharide polymers include quaternary nitrogen-containing cellulose ethers (e.g. as described in US3962418 from L'Oréal), and copolymers of etherified cellulose and starch (e.g. as described in US3958581 from L'Oréal).

A particularly suitable type of cationic polysaccharide polymer that can be used is a cationic guar gum derivative, such as guar hydroxypropyltrimethylammonium chloride (commercially available from Solvay in their JAGUAR trade named series). Examples of such materials are JAGUAR C13S, JAGUAR C14, JAGUAR C15, JAGUAR C17 and JAGUAR C16 Jaguar CHT and JAGUAR C162.

Mixtures of any of the above cationic polymers may be used.

Cationic polymer may be present in a shampoo composition of the invention at levels of from 0.01 to 5 wt.-%, preferably from 0.05 to 1 wt.-%, more preferably from 0.08 to 0.5 wt.-% by total weight of cationic polymer based on the total weight of the composition.

In at least one embodiment, the cationic polymers have a number average molecular weight of at least about 5000 g/mol, typically from 10000 g/mol to 10 million g/mol and are selected from the group consisting of: copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. Other suitable spacer monomers include vinyl esters, vinyl alcohol, maleic anhydride, propylene glycol, and ethylene glycol. Preferred cationic polymers are cationic celluloses, cationic starches, and cationic guar gums. Commercially available cationic guar polymers are e.g. Jaguar^{®} from Solvay.

In at least one embodiment, the composition comprises a preservative or preservative system. Examples of suitable preservatives include benzyl alcohol, piroctone olamine, phenoxyethanol, parabens, pentanediol, benzoic acid/sodium benzoate, sorbic acid/potassium sorbate, and other organic acids used to provide antimicrobial protection. Preservation boosting ingredients include anisic acid, lactic acid, sorbitan caprylate, ethylhexylglycerin, caprylyl glycol, octanediol, and similar substances. In at least one embodiment, the composition comprises 0.01 to 5 wt.-%, particularly preferably from 0.05 wt.-% to 1 wt.-% of at least one preservative. Suitable preservatives are the substances listed in the International Cosmetic Ingredient Dictionary and Handbook, 9th Edition with the function "preservatives". In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the composition comprises a preservative selected from the group consisting of cetyltrimethyl ammoniumchloride, cetylpyridinium chloride, benzethonium chloride, diisobutylethoxyethyldimethyl benzylammoniumchloride, sodium N-lauryl sarcosinate, sodium-N-palmethylsarcosinate, Lauroylsarcosine, N-myristoylglycine, potassium-N-laurylsarcosine, trimethylammoniumchloride, sodium aluminium chlorohydroxylactate, triethylcitrate, tricetylmethylammoniumchloride, 2,4,4'-trichloro-2'-hydroxydiphenylether (Triclosan), phenoxyethanol, 1,5-pentandiol, 1,6-hexandiol, 3,4,4'-trichlorocarbanilide (Triclocarban), diaminoalkylamide, L-lysine hexadecylamide, heavy metal citrate salts, salicylate, piroctose, zinc salts, pyrithione and its heavy metal salts, zinc pyrithione, zinc phenol sulfate, farnesol, ketoconazol, oxiconazol, bifonazole, butoconazole, cloconazole, clotrimazole, econazole, enilconazole, fenticonazole, isoconazole, miconazole, sulconazole, tioconazole, fluconazole, itraconazole, terconazole, naftifine, terbinafine, selenium disulfide, Octopirox^{®}, methylchloroisothiazolinone, methylisothiazolinone, methyldibromo glutaronitrile, AgCI, chloroxylenol, sodium salts of diethylhexylsulfosuccinate, sodiumbenzoate, phenoxyethanol, benzylalkohol, phenoxyisopropanol, paraben, such as butyl-, ethyl-, methyl- und propylparaben, and their salts, pentandiol, 1,2-octanediol, ethylhexylglycerinw, benzylalcohol, sorbic acid, benzoic acid, lactic acid, imidazolidinyl urea, diazolidinyl urea, dimethylol dimethyl hydantoin (DMDMH), sodium salts of hydroxymethyl glycinate, hydroxyethylglycine of sorbic acid and combinations thereof. In at least one embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one embodiment, the composition is substantially free of parabens.

The shampoo compositions of the present invention can be in the form of rinse-off products or 'dry shampoo' products, can be opaque or transparent, and can be formulated in a wide variety of product forms, including creams, gels, emulsions, mousses and sprays. Preferably, the hair cleanser composition of the present invention is in the form of a rinse-off product.

In at least one embodiment, the shampoo composition is silicone-free.

In at least one embodiment, the shampoo composition is sulfate-free.

In at least one embodiment, the shampoo composition comprises:
(i) from 0.2 wt.-% to 2.0 wt.-% of one or more oligoester ammonium salts (OAS) as described herein;
(ii) from 5 wt.-% to 20 wt.-% of one or more anionic surfactants, wherein the composition comprises at least one alkyl sulfate or alkyl ether sulfate;
(iii) at least 50 wt.-% water; and
(iv) at least one further cosmetically acceptable component (F) selected from the group consisting of silicone, cationic polymer, rheology modifiying agent, and an amphoteric or zwitterionic surfactant.

A preferred embodiment of the shampoo composition relates to a shampoo composition comprising:
(i) from 0.2 wt.-% to 2.0 wt.-% of one or more oligoester ammonium salts (OAS) as described herein;
(ii) from 5 wt.-% to 20 wt.-% of one or more anionic surfactants, wherein the composition comprises at least one alkyl sulfate or alkyl ether sulfate;
(iii) at least 50 wt.-% water; and
(iv) at least one further component selected from the group consisting of silicone, cationic polymer, rheology modifiying agent, and an amphoteric or zwitterionic surfactant;
(v) at least one further cosmetically acceptable component (F).

A preferred embodiment of the shampoo composition relates to a shampoo composition consisting of:
(i) from 0.2 wt.-% to 2.0 wt.-% of one or more oligoester ammonium salts (OAS) as described herein;
(ii) from 5 wt.-% to 20 wt.-% of one or more anionic surfactants, wherein the composition comprises at least one alkyl sulfate or alkyl ether sulfate;
(iii) at least 50 wt.-% water; and
(iv) at least one further component selected from the group consisting of silicone, cationic polymer, rheology modifiying agent, and an amphoteric or zwitterionic surfactant;
(v) at least one further cosmetically acceptable component (F) selected from the group consisting of conditioning agents, such as hydrolysed collagen, vitamin E, or panthenol, panthenyl ethyl ether, hydrolysed keratin, proteins, plant extracts, nutrients; and emollients such as PPG-3 myristyl ether, trimethyl pentanol hydroxyethyl ether; hair-fixative polymers such as amphoteric fixative polymers, cationic fixative polymers, anionic fixative polymers, nonionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents; hair oxidizing (bleaching) agents, such as hydrogen peroxide, perborate and persulfate salts; hair reducing agents such as the thioglycolates; perfumes; and sequestering agents, such as disodium ethylene-diamine tetraacetate; ultraviolet and infrared screening and absorbing agents such as octyl salicylate; and anti-dandruff agents such as zinc pyrithione and salicylic acid.

The preparation of the oligoester ammonium salt of general formula (I) can be achieved by methods known to those skilled in the art, for example, by
(i) firstly contacting the components of a reaction mixture (a) to (d) under increased temperature of 80 to 220°, in particular 150 to 210°C by continuously removing of reaction water:
   0.5 to 3.0 molar equivalents of a diethanolamine compound represented by the formula (a) 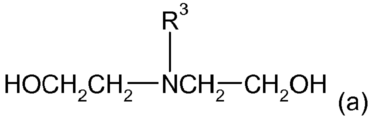 wherein R³ is a linear or branched C₁-C₆-alkyl group, preferably a linear or branched C₁-C₄-alkyl group, more preferably is methyl or ethyl;
   0.5 to 1.5 molar equivalents of a dicarboxylic acid represented by the formula (b) 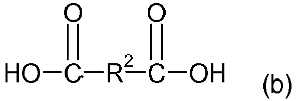
   wherein R² is a linear or branched C₁-C₁₀-alkylene group or a linear or branched C₂-C₁₀-alkenylene group, preferably is a C₂-C₈-alkylene, more preferably is a C₄-alkylene group;
   0.5 to 1.5 molar equivalents of an organic triol represented by the formula (c) 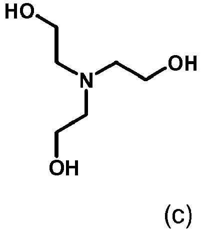
   1.0 molar equivalent of a monocarboxylic acid represented by formula (d)

      R¹-COOH (d)

      wherein R¹ is a linear or branched C₁₂-C₂₄-alkyl or linear or branched C₁₂-C₂₄-alkenyl group, preferably a linear or branched C₁₂-C₂₂-alkyl or linear or branched C₁₂-C₂₂-alkenyl group, more preferably is C₂₀-C₂₂-alkyl;
(ii) reacting the oligoester product of step (i) with a quaternization agent (e), preferably from the group of commonly known alkylating agents such as dimethyl sulfate, diethyl sulfate and alkyl halides;
   and then
(iii) optionally removing and purifying the at least one oligoester ammonium salt.

Suitable alkylating agents are, for example, dimethyl sulfate, diethyl sulfate, dimethyl carbonate, diethyl carbonate, and alkyl halides, like methyl chloride, ethyl chloride, methyl bromide, ethyl bromide, methyl iodide or ethyl iodide.

The contacting step is carried out under conditions sufficient to produce oligoester amine, wherein n is an integer from 1 to about 30. The components of the reaction mixture can be contacted either sequentially or simultaneously. Preferably, the component (a), said dicarboxylic acid (b), said polyol (c) and said monocarboxylic acid (d) are contacted in the molar ratios described. Preferably, the contacting is carried out in the presence of an esterification catalyst, such as, a Brönstedt or Lewis acid catalyst, or a metal catalyst. Examples are hypophosphorous acid, methane sulfonic acid, p-toluene sulfonic acid, titanium tetrabutylate or Fascat^{®}.

The invention also relates to a multiple-part kit of hair cleaning and hair conditioning compositions. The term "kit" includes items that are either sold or packaged together. The multiple-part kit may be distributed to end users through salons, but one aspect of the invention involves distributing the kits to consumers through retail sales channels such as drugstores, cosmetic stores and on-line stores. The kit comprises separate compartments with formulations for a shampoo and a conditioning treatment. The term "compartment" refers to any receptacle, regardless of shape, material or closure, which serves a containing function. The term includes the interior of a tube, sack, can, tub, bottle, packet, envelope or other vessel. The components of the multiple-part kit may be contained in a single receptacle, or may be divided amongst multiple receptacles. The multiple-part kit can additionally comprise a compartment with a composition to colour the hair and/or a composition to moisturize and maintain the quality of the treated hair.

For bleached or coloured hair, additional compartments in the multiple-part kit are advantageous. There is a high need for a hair cleaning and hair conditioning kit, that is easy to use and that provides a consumer specialized care regimen to preserve the condition of the hair. The invention provides all this in one multiple-part kit with all the components needed to maintain the condition of the hair for several weeks. The kit may include at least one compartment containing a pre-treatment composition.

### EXAMPLES

The invention is further illustrated by the following non-limiting examples.

### Example 1: Preparation of oligoester precursor

Methyl diethanol amine (component (a), 223.4 g, 1.875 mol), behenic acid (component (d), 510.9 g, 1.5 mol), adipic acid (component (b), 219.2 g, 1.5 mol) and triethanolamine (component (c), 138.16 g, 1.5 mol) were charged in a 2 L flask equipped with a stirrer, distillation apparatus and thermometer under an atmosphere of nitrogen. The reaction mixture was heated to 160°C and the distillation of reaction water started. The temperature was increased to 210°C when the head temperature decreased. The reaction water was continually removed from the reaction mixture by distillation at 160 - 210°C over a period of 7 h.

The product (874 g) was obtained as brown solid (acid value: 7.4 mg KOH/g; Bas-N: 4.38 %; Mₙ = 1130 g/mol, M_{w} = 1476 g/mol).

### Example 2: Oligoester ammonium salt

The oligoester of Example 1 (150.0 g) was charged in a 500 mL flask equipped with a stirrer, dropping funnel and thermometer under an atmosphere of nitrogen and heated to 85 °C. Dimethyl sulfate (29.6 g, 0.24 mol, 0.5 equivalents/ nitrogen atom in the oligoester) was added dropwise over a period of 30 minutes. The reaction mixture was stirred for 5 h at 90 °C. The product (169 g) was obtained as light brown solid (Bas.-N: 2.10 %).

### Example 3: Oligoester ammonium salt

The oligoester of Example 1 (150.0 g) was charged in a 500 mL flask equipped with a stirrer, dropping funnel and thermometer under an atmosphere of nitrogen and heated to 85°C. Dimethyl sulfate (58.0 g, 0.46 mol, 0.98 equivalents/ nitrogen atom in the oligoester) was added dropwise over a period of 45 minutes. The reaction mixture was stirred for 5 h at 90 °C. The product (197 g) was obtained as light brown solid (Bas.-N: 0.08 %).

### Example 4: Preparation of oligoester precursor

Methyl diethanol amine (119.2 g, 1.0 mol), behenic acid (510.9 g, 1.5 mol), adipic acid (219.2 g, 1.5 mol) and triethanolamine (223.8 g, 1.5 mol) were charged in a 2 L flask equipped with a stirrer, distillation apparatus and thermometer under an atmosphere of nitrogen. The reaction mixture was heated to 160 °C for 3 h and reaction water was removed from the reaction mixture by distillation. Afterwards the reaction mixture was heated to 200 - 205 °C for 8 h and reaction water was removed from the reaction mixture by distillation. The product (963 g) was obtained as brown wax (acid value: 1.7 mg KOH/g; Bas-N: 3.49 %; Mₙ = 1756 g/mol, M_{w} = 2139 g/mol).

### Example 5 Oligoester ammonium salt (OAS)

The oligoester of Example 4 (300.0 g) was charged in a 1 L flask equipped with a stirrer, dropping funnel and thermometer under an atmosphere of nitrogen and heated to 85 °C. Dimethyl sulfate (92.2 g, 0.73 mol, 0.98 equivalents/ nitrogen atom in the Oligoester) was added dropwise over a period of 20 minutes. During the addition, the resulting product mixture became highly viscous and butyl diglycol (98.1 g) was added. The reaction mixture was stirred for 5 h at 85 °C. The product (400 g, 80 % active content) was obtained as brown wax (Bas.-N: 0.01 %).

### Example 6: Oligoester ammonium salt

The oligoester of Example 4 (330.0 g) was charged in a 1 L flask equipped with a stirrer, dropping funnel and thermometer under an atmosphere of nitrogen and heated to 85 °C. Dimethyl sulfate (51.8 g, 0.41 mol, 0.5 equivalents/ nitrogen atom in the oligoester) was added dropwise over a period of 20 minutes. During the addition, the resulting product mixture became highly viscous and butyl diglycol (42.4 g) was added. The reaction mixture was stirred for 5 h at 85 °C. The product (380 g, 90 % active content) was obtained as brown wax (Bas.-N: 1.31 %).

### Example 7: Preparation of oligoester precursor

Methyl diethanol amine (238.4 g, 2.0 mol), behenic acid (340.4 g, 1.0 mol), adipic acid (219.2 g, 1.5 mol) and triethanolamine (149.2 g, 1.0 mol) were charged in a 2 L flask equipped with a stirrer, distillation apparatus and thermometer under an atmosphere of nitrogen. The reaction mixture was heated to 160 °C for 2 h and reaction water was removed from the reaction mixture by distillation. Afterwards the reaction mixture was heated to 200 - 205 °C for 8 h and reaction water was removed from the reaction mixture by distillation. The product (898 g) was obtained as brown wax (acid value: 3.4 mg KOH/g; Bas-N: 5.19 %; Mₙ = 1433 g/mol; M_{w} = 1575 g/mol).

### Example 8: Oligoester ammonium salt

The oligoester of Example 7 (150.0 g) was charged in a 500 mL flask equipped with a stirrer, dropping funnel and thermometer under an atmosphere of nitrogen and heated to 85 °C. Dimethyl sulfate (68.7 g, 0.54 mol, 0.98 equivalents/ nitrogen atom in the oligoester) was added dropwise over a period of 45 minutes. During the addition, the resulting product mixture became highly viscous and butyl diglycol (54.7 g) was added. The reaction mixture was stirred for 5 h at 85 °C. The product (252 g, 80 % active content) was obtained as brown wax (Bas.-N < 0.01 %).

### Example 9: Oligoester ammonium salt

The oligoester of Example 7 (150.0 g) was charged in a 500 mL flask equipped with a stirrer, dropping funnel and thermometer under an atmosphere of nitrogen and heated to 85 °C. Dimethyl sulfate (35.3 g, 0.28 mol, 0.5 equivalents/ nitrogen atom in the oligoester) was added dropwise over a period of 20 minutes. During the addition, the resulting product mixture became highly viscous and butyl diglycol (46.0 g) was added. The reaction mixture was stirred for 5 h at 85 °C. The product (218 g, 80 % active content) was obtained as brown wax (Bas.-N = 2.55 %).

### Example 10: Preparation of oligoester precursor

Methyl diethanol amine (238.4 g, 2.0 mol), behenic acid (681.2 g, 2.0 mol), adipic acid (146.1 g, 1.0 mol) and triethanolamine (149.2 g, 1.0 mol) were charged in a 2 L flask equipped with a stirrer, distillation apparatus and thermometer under an atmosphere of nitrogen. The reaction mixture was heated to 170 °C for 2 h and reaction water was removed from the reaction mixture by distillation. Afterwards the reaction mixture was heated to 190 °C for 1 h, then to 200 °C for 1 h and then to 210 °C for 6 h. During heating, reaction water was continually removed from the reaction mixture by distillation. The product (1087 g) was obtained as brown wax (Acid value: 2.8 mg KOH/g; Bas-N: 3.55 %; Mₙ = 1065 g/mol; M_{w} = 1359 g/mol).

### Example 11: Oligoester ammonium salt

The oligoester of Example 10 (150.0 g) was charged in a 500 mL flask equipped with a stirrer, dropping funnel and thermometer under an atmosphere of nitrogen and heated to 60 °C. Dimethyl sulfate (47.0 g, 0.37 mol, 0.98 equivalents/ nitrogen atom in the oligoester) was added dropwise over a period of 30 minutes. During the addition, the resulting product mixture became highly viscous and butyl diglycol (46.75 g) was added. The reaction mixture was heated to 80 °C and stirred at this temperature for 0.5 h. Subsequently the reaction mixture was heated to 90 °C and stirred at this temperature for 4.5 h. The product (212 g, 80 % active content) was obtained as brown solid (Bas.-N = 0.03 %).

### Example 12: Preparation of oligoester precursor

Methyl diethanol amine (178.8 g, 1.5 mol), behenic acid (340.6 g, 1.0 mol), adipic acid (219.2 g, 1.5 mol) and triethanolamine (223.79 g, 1.5 mol) were charged in a 2 L flask equipped with a stirrer, distillation apparatus and thermometer under an atmosphere of nitrogen. The reaction mixture was heated to 170 °C for 1 h and reaction water was removed from the reaction mixture by distillation. Afterwards the reaction mixture was heated to 190 °C for 1 h, then to 200 °C for 1 h and then to 210 °C for 4 h. During heating, reaction water was continuously removed from the reaction mixture by distillation. The product (774 g) was obtained as brown wax (Acid value: 4.3 mg KOH/g; Bas-N: 4.41 %; Mₙ = 1476 g/mol; M_{w} = 2130 g/mol).

### Example 13: Oligoester ammonium salt

The oligoester of Example 12 (200.0 g) was charged in a 500 mL flask equipped with a stirrer, dropping funnel and thermometer under an atmosphere of nitrogen and heated to 60 °C. Dimethyl sulfate (77.0 g, 0.61 mol, 0.98 equivalents/ nitrogen atom in the oligoester) was added dropwise over a period of 45 minutes. During the addition, the resulting product mixture became highly viscous and butyl diglycol (69.2 g) was added. The reaction mixture was heated to 80 °C and stirred at this temperature for 1 h. Afterwards the temperature was increased to 90 °C and the reaction mixture was stirred at this temperature for additional 4 h. The product (323 g, 80 % active content) was obtained as brown solid (Bas.-N = 0.05 %).

### Example 14: Preparation of oligoester precursor

Methyl diethanol amine (178.8 g, 1.5 mol), behenic acid (170.3 g, 0.5 mol), adipic acid (109.6 g, 0.75 mol) and triethanolamine (37.3 g, 0.25 mol) were charged in a 1 L flask equipped with a stirrer, distillation apparatus and thermometer under an atmosphere of nitrogen. The reaction mixture was heated to 170 °C for 2 h and reaction water was removed from the reaction mixture by distillation. Afterwards the reaction mixture was heated to 190 °C for 1 h and then to 200 °C for 3 h. During heating, reaction water was continually removed from the reaction mixture by distillation. The product (422 g) was obtained as brown solid (Acid value: 2.9 mg KOH/g; Bas-N: 4.99 %; Mₙ = 1176 g/mol; M_{w} = 1380 g/mol).

### Example 15: Oligoester ammonium salt

The oligoester of Example 14 (150.0 g) was charged in a 500 mL flask equipped with a stirrer, dropping funnel and thermometer under an atmosphere of nitrogen and heated to 60 °C. Dimethyl sulfate (66.1 g, 0.52 mol, 0.98 equivalents/ nitrogen atom in the oligoester) was added dropwise over a period of 45 minutes. During the addition, the resulting product mixture became highly viscous and butyl diglycol (54.0 g) was added. The reaction mixture was heated to 90 °C and stirred at this temperature for 5 h. The product (354 g, 80 % active content) was obtained as brown solid (Bas.-N = 0.03 %).

### Example 16: Preparation of oligoester precursor

Methyl diethanol amine (89.4 g, 0.75 mol), behenic acid (340.6 g, 1.0 mol), adipic acid (109.6 g, 0.75 mol) and triethanolamine (223.8 g, 1.50 mol) were charged in a 2 L flask equipped with a stirrer, distillation apparatus and thermometer under an atmosphere of nitrogen. The reaction mixture was heated to 170 °C for 1 h and reaction water was removed from the reaction mixture by distillation. Afterwards the reaction mixture was heated to 190 °C for 3 h and then to 200 °C for 1.5 h. During heating, reaction water was continually removed from the reaction mixture by distillation. The product (690 g) was obtained as brown wax (Acid value: 3.1 mg KOH/g; Bas-N: 4.20 %).

### Example 17: Oligoester ammonium salt (OAS)

The oligoester of Example 16 (150.0 g) was charged in a 500 mL flask equipped with a stirrer, dropping funnel and thermometer under an atmosphere of nitrogen and heated to 60 °C. Dimethyl sulfate (55.6 g, 0.44 mol, 0.98 equivalents/ nitrogen atom in the oligoester) was added dropwise over a period of 45 minutes. During the addition, the resulting product mixture became highly viscous and butyl diglycol (51.4 g) was added. The reaction mixture was heated to 80 °C and stirred at this temperature for 1 h. Afterwards the temperature was increased to 90 °C and the reaction mixture was stirred at this temperature for additional 4 h. The product (235 g, 80 % active content) was obtained as brown solid (Bas.-N = 0.07 %).

### Example 18: Preparation of oligoester precursor

Methyl diethanol amine (178.8 g, 1.5 mol), behenic acid (170.3 g, 0.5 mol), sebacic acid (220.8 g, 0.75 mol) and triethanolamine (111.9 g, 0.75 mol) were charged in a 2 L flask equipped with a stirrer, distillation apparatus and thermometer under an atmosphere of nitrogen. The reaction mixture was heated to 170 °C for 1 h and reaction water was removed from the reaction mixture by distillation. Afterwards the reaction mixture was heated to 190 °C for 2 h and then to 200 °C for 2 h. During heating, reaction water was continually removed from the reaction mixture by distillation. The product (530 g) was obtained as brown solid (Acid value: 2.9 mg KOH/g; Bas-N: 4.68 %; Mₙ = 1550 g/mol; M_{w} = 2310 g/mol).

### Example 19: Oligoester ammonium salt

The oligoester of Example 18 (150.0 g) was charged in a 500 mL flask equipped with a stirrer, dropping funnel and thermometer under an atmosphere of nitrogen and heated to 60 °C. Dimethyl sulfate (62.0 g, 0.49 mol, 0.98 equivalents/ nitrogen atom in the oligoester) was added dropwise over a period of 45 minutes. During the addition, the resulting product mixture became highly viscous and butyl diglycol (53.0 g) was added. The reaction mixture was heated to 80 °C and stirred at this temperature for 1 h. Afterwards the temperature was increased to 90 °C and the reaction mixture was stirred at this temperature for additional 4 h. The product (236 g, 80 % active content) was obtained as brown solid (Bas.-N = 0.05 %).

### Examples 21 to 59: Preparation of hair conditioner compositions

The hair conditioner compositions of examples 21 to 59 are prepared by mixing the components, as listed in Table 1. All cosmetic compositions are creamy white liquids with appearance and physical properties similar to commercially available rinse-off hair conditioner products. The pH-values of the formulations are adjusted to 3.5 to 4.5. INCI names are provided for each ingredient. Xiameter PMX-200 is e.g. a silicone fluid (Dimethicone). CE = comparative example.

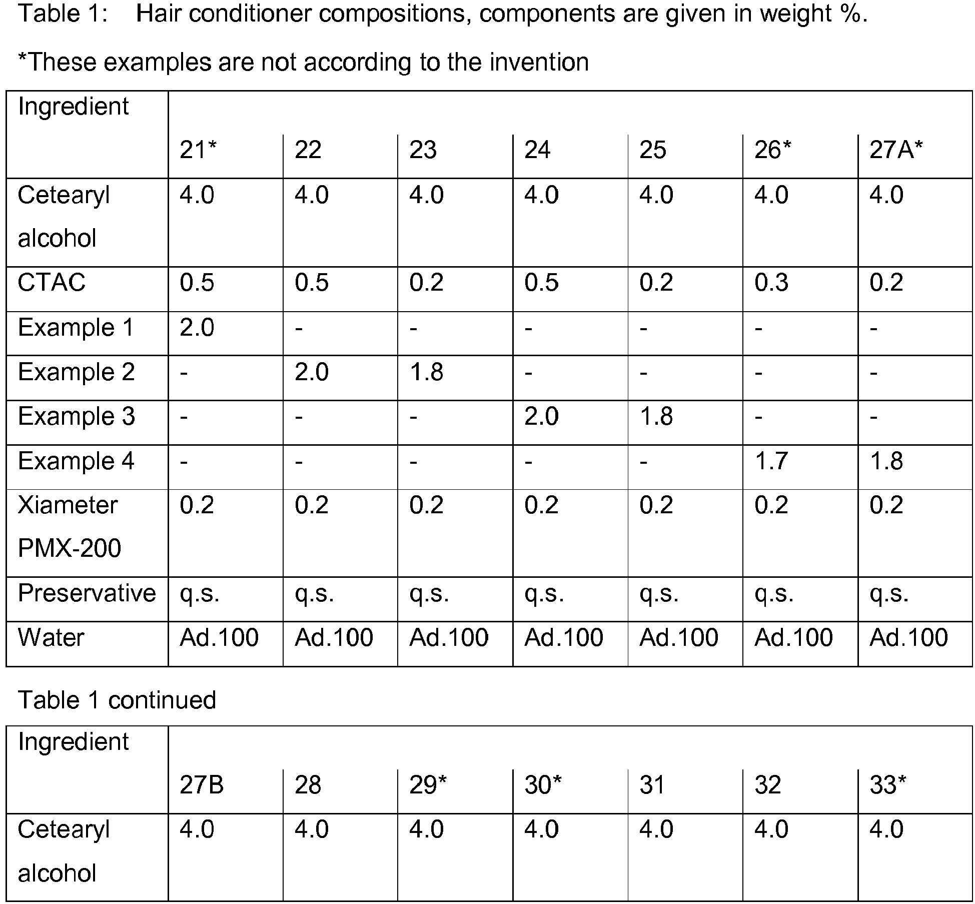

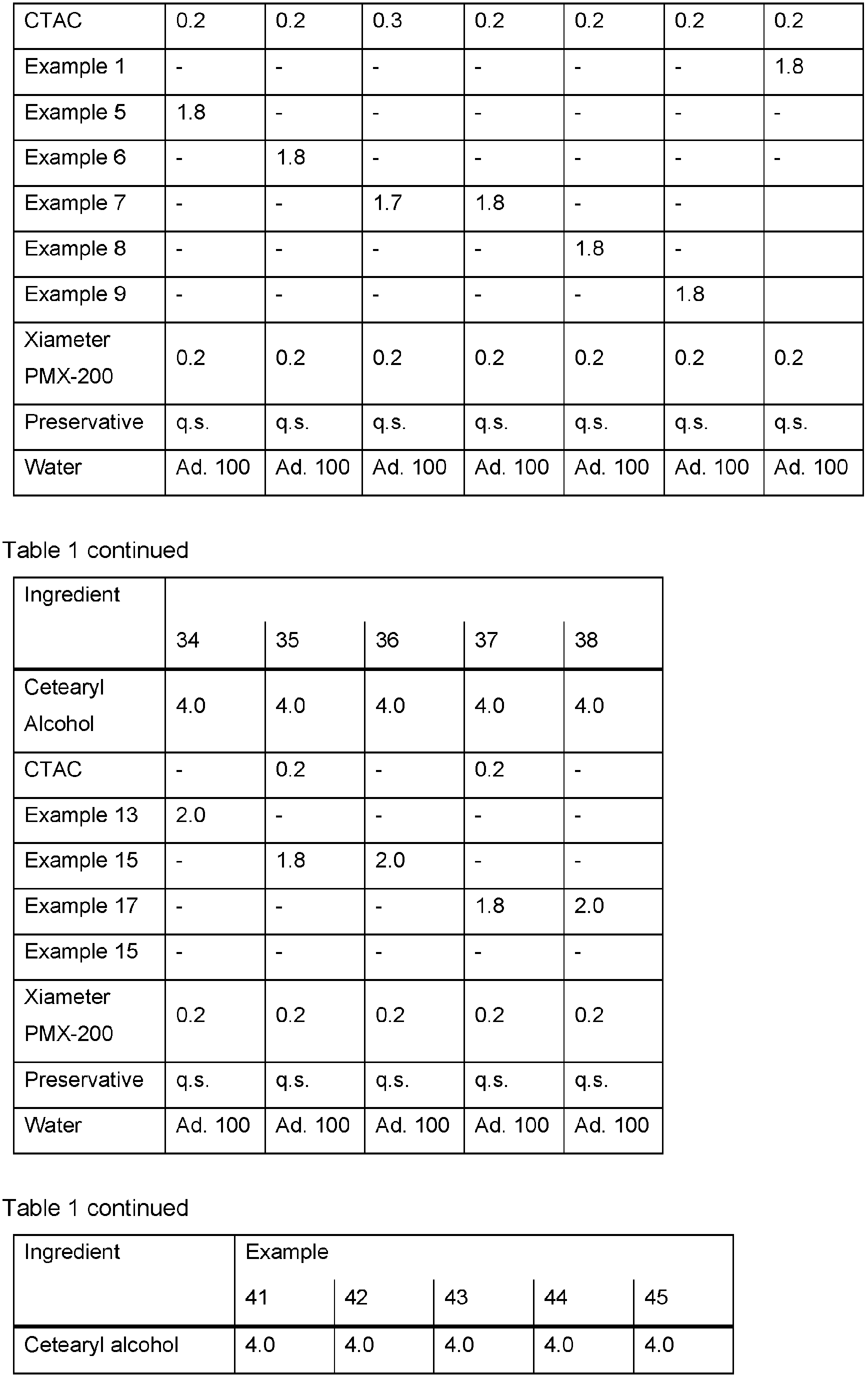

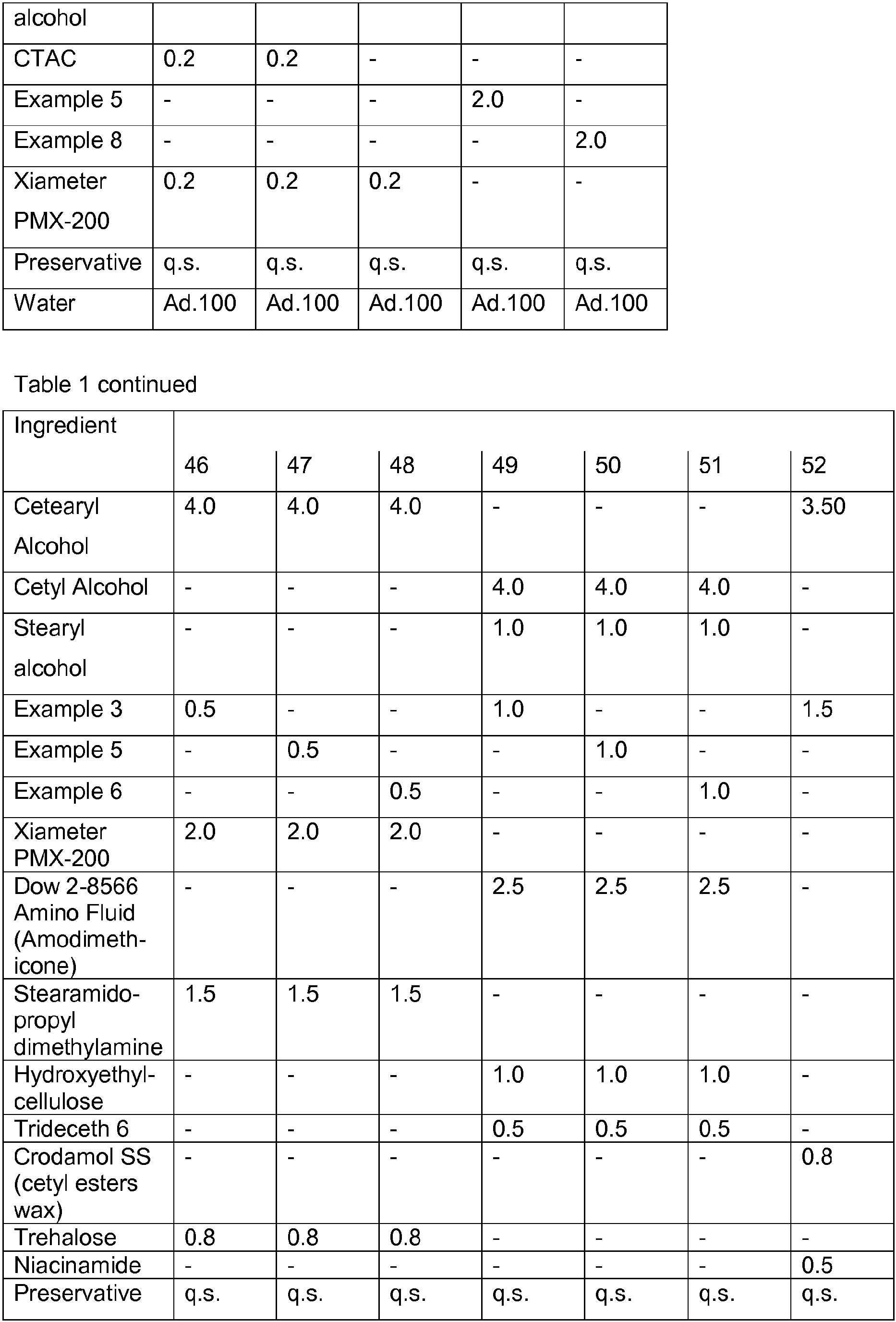

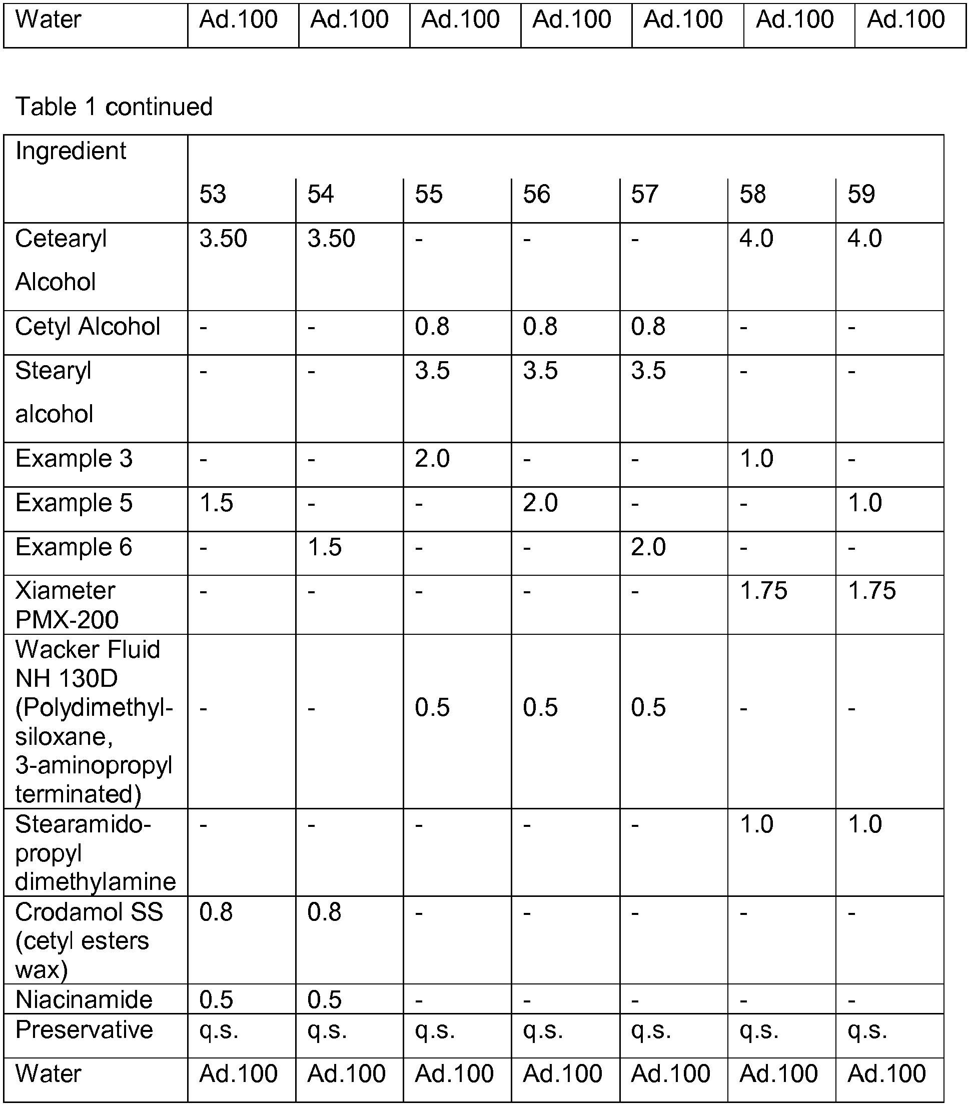

### Example 60: testing of the conditioner compositions

The studies are conducted with hair swatches (using dark brown, straight European hair tresses from Kerling, 15 cm long, ca. 2.6 g hair each). Virgin hair (not chemically treated) and damaged (4 times bleached) hair are used.

Hair swatches are pre-treated (base wash with a 14 % by weight sodium lauryl ether sulfate (SLES) solution), and then treated with one of the conditioner as described in examples 21 to 59. The following steps are used:
a) applying a shampoo formulation onto the hair;
b) washing the hair with the shampoo formulation;
c) removing the shampoo composition from the hair;
d) applying the conditioner composition of examples 21 to 59 onto the hair;
e) removing said conditioner composition from the hair.

The combing forces in wet and dry state are measured using a typically used instrument (Diastron (UK) MTT175), wet combing after rinsing off the conditioner, and dry combing after at least 12 hours of drying time in air at 22 °C. The hair swatches are pre-combed three times before the measurement.

As benchmarks, analogous conditioner compositions are used containing cetrimmonium chloride (CTAC) or behentrimonium chloride (BTAC) at 2 % by weight, active level, as listed in Table 2. The Table 3 then presents the wet (average) combing force (gmf force) for hair treated with selected conditioner compositions of examples 21 to 59 (see formulations in Table 1).

**Table 2: Formulations of comparative Example (CE) hair conditioner compositions (active levels of ingredients in weight %, Silicone from Dow Corning (DC200)).**

| Ingredient | Example (CE1) | Example (CE2) |
|---|---|---|
| Cetearyl Alcohol | 4.0 | 4.0 |
| CTAC | 2.0 | - |
| BTAC (Behentrimonium Chloride) | - | 2.0 |
| Xiameter PMX-200 | 0.2 | 0.2 |
| Preservative | q.s. | q.s. |
| Water | Ad. 100 | Ad. 100 |

**Table 3: Wet combing force results of hair treated with conditioner compositions (Examples 28, 44, 47, 48, 50, 56) compared to benchmark formulations (CE1 or CE2).**

| Example | wet combing force, virgin hair (average), gmf | wet combing force, damaged hair (average), gmf |
|---|---|---|
| CE1 | - | 12.1 |
| CE2 | 10.7 | - |
| Example 28 | 9.7 | |
| Example 44 | 8.8 | 11.8 |
| Example 47 | 10.2 | 9.2 |
| Example 48 | 9.6 | 9.6 |
| Example 50 | - | 10.2 |
| Example 56 | 8.6 | - |

Table 4 presents the dry (maximum) combing force (gmf force) for hair treated with selected conditioner composition of Example 44. As benchmark, comparative example CE1 is used (formulation in Table 2).

**Table 4: Dry combing force results of hair treated with conditioner composition (Example 44), compared to benchmark formulation CE1.**

| Example | dry combing force, virgin hair (max), gmf | dry combing force, damaged hair (max), gmf |
|---|---|---|
| CE1 | 140.8 | 117.2 |
| Example 44 | 67.3 | 55.3 |

The dry hair after treatments with conditioner compositions of examples 21 to 59 showed good tactile results and led to nice hair appearance. In particular, conditioner compositions of example 44 produced an effect of very soft hair.

After using the method of treating hair with the conditioning composition, comprising an optional pre-treatment with silicone-free shampoo and a conditioner treatment, the hair probes are tested for hair shine (using a classical testing with Samba Hair System, from Bossa Nova Tech). This measurement technique allows for quantitative evaluation of the light intensity reflected from hair swatches mounted on a drum in a half-circle arrangement.

Results of the shine measurements are illustrated in Table 5, for hair swatches treated with compositions of examples 25, 26, 28, 44, which in general show stronger shine than the prior art formulation CE1.

**Table 5: Hair shine results for swatches treated with conditioner compositions**

| Example | hair shine, virgin hair | hair shine, damaged hair |
|---|---|---|
| CE 1 | 15.5 | 6.8 |
| Example 25 | 16.7 | - |
| Example 26 | 17.5 | - |
| Example 28 | 17.5 | - |
| Example 44 | 15.6 | - |

### Evaluation of hair volume and frizz

Hair volume was measured by digital image analysis using ImageJ software. Hair swatches were mounted on a holder in front of a light box, images were taken and evaluated. For each measurement, nine images were used, and the swatch area corresponding to the continuous dark signal intensity was measured. Volume increase or reduction was compared to that of an untreated hair swatch. The frizz component of hair volume was calculated with the ImageJ software as the area in the image on the perimeter of the continuous hair swatch, in which single fibres were visible. The results are reported as volume or frizz change in % of the volume/ frizz of the untreated hair (increase - positive; reduction - negative).

The following formulations were tested:

**Table 6:**

| Ingredient | Example 61 | Example 62 | CE 1 | CE 3 |
|---|---|---|---|---|
| Cetearyl Alcohol | 4.0 | 4.0 | 4.0 | 4.0 |
| Example 5 | 1.7 | 1.7 | - | - |
| CTAC | 0.3 | 0.3 | 2.0 | 2.0 |
| Xiameter PMX-200 | 0.2 | - | 0.2 | - |
| Preservative | q.s. | q.s. | q.s. | q.s. |
| Water | Ad. 100 | Ad. 100 | Ad. 100 | Ad. 100 |

**Table 7: Hair volume changes, virgin European hair:**

| Product tested | CE 3 | CE 1 | Example 62 | Example 61 |
|---|---|---|---|---|
| Volume change, % | +11.3 | +4.2 | +10.1 | -2.1 |

It is clear and consistent with the knowledge in the field that hair cosmetics without silicone increase hair volume (CE 3 and Example 62). To the contrary, products with silicone reduce hair volume to give the effect of smooth and sleek hair. Volume reduction by using the product with Example 5 is stronger than for a conventional conditioner (CE 1).

**Table 8: Hair frizz changes, virgin European hair:**

| Product tested | CE 1 | Example 62 | Example 61 |
|---|---|---|---|
| Frizz change, % | -33 | -49.3 | -84.5 |

Hair frizz is a component of hair volume, and is generally unwelcome among consumers, as it decreases hair gloss and makes hair less manageable. Typical conditioners are able to reduce hair frizz, and those with added silicone normally have a stronger effect. Material of Example 5 reduces hair frizz stronger than a benchmark conditioner CE 1 (with silicone). Whether silicone-free or with silicone, Example 5 has a much stronger effect in frizz reduction, and can be used in smoothing, frizz reducing products. Hair gloss of frizz-free hair is also expected to be higher.

### Evaluation of hair smoothness and repair

Hair smoothness and repair was measured on various types of hair using Diastron (UK) MTT 175 tester with the friction accessory. Friction coefficient for hair when moved in the root-to-tip direction under external weight is interpreted as smoothness, and in the opposite direction (tip-to-root) as hair repair.

The following formulations were tested:

**Table 9:**

| Ingredient | Example 61 | CE 1 | CE 2 |
|---|---|---|---|
| Cetearyl Alcohol | 4.0 | 4.0 | 4.0 |
| Example 5 | 1.7 | - | - |
| CTAC | 0.3 | 2.0 | - |
| BTAC | - | - | 2.0 |
| Xiameter PMX-200 | 0.2 | 0.2 | 0.2 |
| Preservative | q.s. | q.s. | q.s. |
| Water | Ad. 100 | Ad. 100 | Ad. 100 |

Hair smoothness

The measurement error is +/- 2%. The following results were obtained for friction coefficient for European (virgin and 4 h bleached) and Asian (virgin) hair:

**Table 10:**

| Product tested | CE 1 | CE 2 | Example 61 |
|---|---|---|---|
| Friction coefficient root-to-tip | 0.5838 | 0.5462 | 0.5378 |
| - EU virgin | | | |
| Friction coefficient root-to-tip | - | 0.5728 | 0.5458 |
| - EU bleached | | | |
| Friction coefficient root-to-tip | 0.5856 | - | 0.5650 |
| - Asian virgin | | | |

A lower friction coefficient corresponds to smoother hair, and differences of 0.01 are already significant. The conditioner of Example 61 shows improved performance on that benefit in comparison with typical conditioners (CTAC and BTAC).

### Hair repair

The measurement error is +/- 2%. The following results were obtained for return friction coefficient for European (virgin and 4 h bleached) and Asian (virgin) hair:

**Table 11:**

| Product tested | CE 1 | CE 2 | Example 61 |
|---|---|---|---|
| Friction coefficient tip-to-root | 1.217 | 1.202 | 1.031 |
| - EU virgin | | | |
| Friction coefficient tip-to-root | 1.120 | 1.077 | 1.070 |
| - EU bleached | | | |
| Friction coefficient tip-to-root | 1.155 | - | 1.115 |
| - Asian virgin | | | |

Material of Example 5, when formulated in a hair conditioner, shows a significantly improved benefit of hair repair in comparison with conventional conditioners (CE 1 and CE 2), on all hair types used for the test.

### Evaluation of hair gloss/shine with and without silicone

Glossy hair is among the largest consumer needs when hair care products are considered. A number of commonly known hair glossers are used in the industry, most frequently silicones. However, with the silicone-free product segment increasing, and with more consumers perceiving silicones as environmentally unfriendly, it is important that a hair conditioner without silicone also provides a high level of hair gloss. Hair gloss on the dry swatches was measured with Samba Hair System (Bossa Nova Tech, CA, USA). BNT numbers for hair gloss calculated by the Bossa Nova algorithm are reported.

**Table 12:**

| Product tested | CE 1 | CE 3 | Example 61 | Example 62 |
|---|---|---|---|---|
| EU virgin hair | 15.9 | - | 17.0 | 16.9 |
| EU bleached hair | 6.6 | 6.5 | 7.0 | 7.2 |

Conditioner examples 61 (with silicone) and 62 (silicone-free), both containing the material of Example 5, provide higher shine than a prior-art formulations with cetrimonium chloride.

The following examples present shampoo formulations prepared using oligoester compounds of Examples 1 to 19:

**Example 63: A shampoo composition**

| Ingredient | Active level, % |
|---|---|
| Aqua | To 100 |
| sodium laureth sulfate | 8 |
| sodium lauryl sulfate | 8 |
| cocamide MIPA | 4 |
| glycerin | 3.5 |
| glycol distearate | 3 |
| sodium chloride | 2.5 |
| dimethicone | 2 |
| Pyrus malus extract | 1.5 |
| PPG-5-ceteth-20 | 1.5 |
| salicylic acid | 1 |
| Example 5 | 1 |
| carbomer | 0.4 |
| niacinamide | 0.2 |
| pyridoxine HCI | 0.2 |
| guar hydroxypropyltrimonium chloride | 0.2 |
| Saccharum officinarum extract | 0.2 |
| hydrolyzed soy protein | 0.2 |
| methyl cocoate | 0.15 |
| sodium cocoate | 0.15 |
| Parfum | As needed |
| Preservative | As needed |
| citric acid | As needed, pH 4.5 - 6.5 |
| sodium hydroxide | As needed, pH 4.5 - 6.5 |
| Colorant | As needed |

**Example 64: A shampoo composition**

| Ingredient | Active level, % |
|---|---|
| Aqua | To 100 |
| sodium laureth sulfate | 15 |
| dimethicone | 3 |
| glycol distearate | 3 |
| coco-betaine | 2 |
| guar hydroxypropyltrimonium chloride | 1.5 |
| Example 5 | 1.5 |
| niacinamide | 0.5 |
| cocamide MIPA | 0.5 |
| saccharum officinarum extract | 0.5 |
| sodium chloride | 0.5 |
| hydroxypropyltrimonium hydrolyzed wheat protein | 0.4 |
| sodium cocoate | 0.3 |
| aminopropyl triethoxysilane | 0.25 |
| salicylic acid | 0.25 |
| caprylic/capric triglyceride | 0.2 |
| carbomer | 0.2 |
| disodium cocoamphodiacetate | 0.15 |
| potassium chloride | 0.15 |
| methyl cocoate | 0.1 |
| Parfum | As needed |
| Preservative | As needed |
| citric acid | As needed, pH 4.5 - 6.5 |
| sodium hydroxide | As needed, pH 4.5 - 6.5 |
| Colorant | As needed |

**Example 65: A shampoo composition**

| Ingredient | Active level, % |
|---|---|
| Aqua | To 100 |
| sodium laureth sulfate | 14 |
| coco-betaine | 4 |
| glycerin | 3 |
| glycol distearate | 2.5 |
| dimethicone | 2 |
| Example 8 | 1.5 |
| niacinamide | 0.5 |
| coconut oil | 0.5 |
| saccharum officinarum extract | 0.5 |
| sodium chloride | 0.5 |
| olive fruit oil | 0.4 |
| PPG-5-ceteth-20 | 0.3 |
| trideceth-6 | 0.25 |
| polyquaternium-6 | 0.25 |
| salicylic acid | 0.25 |
| amodimethicone | 0.2 |
| carbomer | 0.2 |
| cetrimonium chloride | 0.15 |
| Parfum | As needed |
| Preservative | As needed |
| citric acid | As needed, pH 4.5 - 6.5 |
| sodium hydroxide | As needed, pH 4.5 - 6.5 |
| Colorant | As needed |

**Example 66: A shampoo composition**

| Ingredient | Active level, % |
|---|---|
| Aqua | To 100 |
| ammonium lauryl sulfate | 16 |
| cocoamidopropyl betaine | 5 |
| sodium chloride | 2.5 |
| niacinamide | 2 |
| Saccharum officinarum extract | 1 |
| Example 8 | 1 |
| hydroxypropyl guar hydroxypropyltrimonium chloride | 0.8 |
| salicylic acid | 0.3 |
| hexylene glycol | 0.1 |
| Parfum | As needed |
| Preservative | As needed |
| citric acid | As needed, pH 4.5 - 6.5 |
| sodium hydroxide | As needed, pH 4.5 - 6.5 |
| Colorant | As needed |

**Example 67: A shampoo composition**

| Ingredient | Active level, % |
|---|---|
| Aqua | To 100 |
| sodium laureth sulfate | 16 |
| glycol distearate | 4 |
| coco-betaine | 2.5 |
| glycerin | 2 |
| sodium chloride | 1.5 |
| dimethicone | 1.5 |
| Example 9 | 1.5 |
| niacinamide | 1 |
| guar hydroxypropyltrimonium chloride | 0.8 |
| cocamide MIPA | 0.5 |
| Saccharum officinarum extract | 0.3 |
| PPG-5-ceteth-20 | 0.2 |
| salicylic acid | 0.2 |
| fumaric acid | 0.15 |
| carbomer | 0.15 |
| pyridoxine HCI | 0.1 |
| Parfum | As needed |
| Preservative | As needed |
| citric acid | As needed, pH 4.5 - 6.5 |
| sodium hydroxide | As needed, pH 4.5 - 6.5 |
| Colorant | As needed |

**Example 68: A shampoo composition**

| Ingredient | Active level, % |
|---|---|
| Aqua | To 100 |
| sodium laureth sulfate | 16 |
| coco-betaine | 4 |
| niacinamide | 2 |
| Ribes nigrum oil | 1.5 |
| cocamide MIPA | 1.5 |
| sodium chloride | 1.5 |
| Example 11 | 1.2 |
| sodium cocoate | 0.8 |
| cocamide MIPA | 0.5 |
| Olea europaea oil (olive) fruit oil | 0.3 |
| PEG-55 propylene glycol oleate | 0.3 |
| PEG-60 hydrogenated castor oil | 0.3 |
| polyquaternium-7 | 0.2 |
| salicylic acid | 0.15 |
| amodimethicone | 0.15 |
| propylene glycol | 0.1 |
| Butyrospermum parkii butter | 0.1 |
| laureth-5 carboxylic acid | 0.1 |
| Parfum | As needed |
| Preservative | As needed |
| citric acid | As needed, pH 4.5 - 6.5 |
| sodium hydroxide | As needed, pH 4.5 - 6.5 |
| Colorant | As needed |

**Example 69: A shampoo composition**

| Ingredient | Active level, % |
|---|---|
| Aqua | To 100 |
| sodium laureth sulfate | 10 |
| disodium cocamphodiacetate | 5 |
| glycerin | 3 |
| glycol distearate | 2 |
| sodium chloride | 1.5 |
| Example 5 | 1.5 |
| cocamide MEA | 1 |
| polyquaternium-10 | 1 |
| salicylic acid | 0.5 |
| carbomer | 0.3 |
| Parfum | As needed |
| Preservative | As needed |
| citric acid | As needed, pH 4.5 - 6.5 |
| sodium hydroxide | As needed, pH 4.5 - 6.5 |
| Colorant | As needed |

**Example 70: A shampoo composition**

| Ingredient | Active level, % |
|---|---|
| Aqua | To 100 |
| sodium laureth sulfate | 12 |
| coco-betaine | 3 |
| cocamide MIPA | 2 |
| sodium chloride | 1.8 |
| Olea europaea (olive) fruit oil | 0.5 |
| Example 9 | 0.5 |
| PPG-5-ceteth-20 | 0.4 |
| PEG-55 propylene glycol oleate | 0.4 |
| PEG-60 hydrogenated castor oil | 0.25 |
| polyquaternium-10 | 0.25 |
| salicylic acid | 0.2 |
| amodimethicone | 0.15 |
| propylene glycol | 0.15 |
| laureth-5 | 0.1 |
| Parfum | As needed |
| Preservative | As needed |
| citric acid | As needed, pH 4.5 - 6.5 |
| sodium hydroxide | As needed, pH 4.5 - 6.5 |
| Colorant | As needed |

**Example 71: A shampoo composition**

| Ingredient | Active level, % |
|---|---|
| Aqua | To 100 |
| sodium laureth sulfate | 14 |
| coco-betaine | 3 |
| laureth-5 carboxylic acid | 2.5 |
| cocamide MIPA | 2 |
| amodimethicone | 1.5 |
| sodium chloride | 1.5 |
| Example 9 | 1 |
| polyquaternium-10 | 0.5 |
| PPG-5-ceteth-20 | 0.5 |
| PEG-55 propylene glycol oleate | 0.3 |
| PEG-60 hydrogenated castor oil | 0.3 |
| coconut oil | 0.2 |
| Parfum | As needed |
| Preservative | As needed |
| citric acid | As needed, pH 4.5 - 6.5 |
| sodium hydroxide | As needed, pH 4.5 - 6.5 |
| Colorant | As needed |

**Example 72: A shampoo composition**

| Ingredient | Active level, % |
|---|---|
| Aqua | To 100 |
| sodium laureth sulfate | 15 |
| coco-betaine | 3 |
| glycerin | 3 |
| glycol distearate | 2.5 |
| sodium chloride | 2 |
| amodimethicone | 2 |
| PPG-5-ceteth-20 | 1.5 |
| dimethicone | 1 |
| Example 11 | 0.8 |
| polyquaternium-6 | 0.4 |
| carbomer | 0.4 |
| arginine | 0.1 |
| glutamic acid | 0.1 |
| trideceth-6 | 0.1 |
| hydroxypropyltrimonium hydrolyzed wheat protein | 0.1 |
| cetrimonium chloride | 0.1 |
| Parfum | Parfum |
| Preservative | As needed |
| citric acid | As needed, pH 4.5 - 6.5 |
| sodium hydroxide | As needed, pH 4.5 - 6.5 |
| Colorant | As needed |

**Example 73: A shampoo composition**

| Ingredient | Active level, % |
|---|---|
| Aqua | To 100 |
| ammonium lauryl sulfate | 15 |
| cocamidopropyl betaine | 3 |
| sodium chloride | 2 |
| hexylene glycol | 1.8 |
| hydroxypropyl guar hydroxypropyltrimonium chloride | 1.5 |
| Example 19 | 1 |
| Parfum | Parfum |
| Preservative | As needed |
| citric acid | As needed, pH 4.5 - 6.5 |
| sodium hydroxide | As needed, pH 4.5 - 6.5 |
| Colorant | As needed |

**Example 74: A shampoo composition**

| Ingredient | Active level, % |
|---|---|
| Aqua | To 100 |
| sodium cocoyl isethionate | 4 |
| sodium lauryl sulfoacetate | 3 |
| disodium laureth sulfosuccinate | 2.5 |
| cocamidopropyl betaine | 2.5 |
| sodium lauroyl sarcosinate | 2.5 |
| glycol distearate | 2 |
| glycereth-26 | 1.8 |
| decyl glucoside | 1.5 |
| hydrogenated coconut acid | 1.4 |
| PPG-5-ceteth-20 | 1.3 |
| divinyldimethicone/dimethicone copolymer | 1.2 |
| sodium chloride | 1.1 |
| amodimethicone | 1 |
| Example 5 | 1 |
| polyquaternium-7 | 1 |
| polyquaternium-10 | 0.7 |
| sodium isethionate | 0.5 |
| PEG-55 propylene glycol oleate | 0.4 |
| propylene glycol | 0.4 |
| carbomer | 0.4 |
| C11-15 pareth-7 | 0.3 |
| glycerin | 0.3 |
| trideceth-12 | 0.25 |
| laureth-9 | 0.2 |
| hydroxypropyltrimonium hydrolyzed wheat protein | 0.1 |
| Parfum | Parfum |
| Preservative | As needed |
| citric acid | As needed, pH 4.5 - 6.5 |
| sodium hydroxide | As needed, pH 4.5 - 6.5 |
| Colorant | As needed |

**Example 75: A shampoo composition**

| Ingredient | Active level, % |
|---|---|
| Aqua | To 100 |
| sodium laureth sulfate | 10 |
| disodium cocoamphodiacetate | 4 |
| disodium laureth sulfosuccinate | 2.5 |
| sodium chloride | 2 |
| glycol distearate | 1.8 |
| zinc pyrithione | 1.5 |
| PPG-5-ceteth-20 | 1 |
| carbomer | 0.6 |
| Example 9 | 0.5 |
| polyquaternium-10 | 0.4 |
| Parfum | Parfum |
| Preservative | As needed |
| citric acid | As needed, pH 4.5 - 6.5 |
| sodium hydroxide | As needed, pH 4.5 - 6.5 |
| Colorant | As needed |

**Example 76: A shampoo composition**

| Ingredient | Active level, % |
|---|---|
| Aqua | To 100 |
| sodium methyl cocoyl taurate | 5 |
| laureth-5 carboxylic acid | 4 |
| sodium chloride | 2 |
| cocamidopropyl betaine | 2 |
| glycerin | 1.8 |
| glycol distearate | 1.5 |
| Example 9 | 1.5 |
| polyquaternium-10 | 1 |
| PPG-5 ceteth-20 | 0.5 |
| sodium lauroyl glutamate | 0.4 |
| propylene glycol | 0.2 |
| PEG-55 propylene glycol oleate | 0.15 |
| Argania spinosa kernel oil | 0.1 |
| Parfum | Parfum |
| Preservative | As needed |
| citric acid, lactic acid | As needed, pH 4.5 - 6.5 |
| sodium hydroxide | As needed, pH 4.5 - 6.5 |
| Colorant | As needed |

**Example 77: A shampoo composition**

| Ingredient | Active level, % |
|---|---|
| Aqua | To 100 |
| sodium laureth sulfate | 10 |
| disodium cocoamphodiacetate | 4 |
| glycol distearate | 2 |
| sodium chloride | 1.5 |
| Example 11 | 1.3 |
| carbomer | 0.4 |
| diaminopyrimidine oxide | 0.3 |
| disodium ricinoleamido MEA sulfosuccinate | 0.3 |
| hexylene glycol | 0.2 |
| panthenol | 0.1 |
| polyquaternium-10 | 0.1 |
| Parfum | Parfum |
| Preservative | As needed |
| citric acid | As needed, pH 4.5 - 6.5 |
| sodium hydroxide, ammonium hydroxide | As needed, pH 4.5 - 6.5 |
| Colorant | As needed |

**Example 78: A shampoo composition**

| Ingredient | Active level, % |
|---|---|
| Aqua | To 100 |
| ammonium lauryl sulfate | 12 |
| cocamidopropyl betaine | 3 |
| sodium chloride | 1.5 |
| Example 15 | 1.3 |
| sodium chloride | 1 |
| alcohol denat. | 0.8 |
| diethyllutidinate | 0.3 |
| hydroxypropyl guar hydroxypropyltrimonium chloride | 0.2 |
| polyquaternium-30 | 0.15 |
| Parfum | Parfum |
| Preservative | As needed |
| citric acid | As needed, pH 4.5 - 6.5 |
| sodium hydroxide | As needed, pH 4.5 - 6.5 |
| Colorant | As needed |

**Example 79: A shampoo composition**

| Ingredient | Active level, % |
|---|---|
| Aqua | To 100 |
| sodium laureth sulfate | 12 |
| PEG-200 hydrogenated glyceryl palmate | 4 |
| disodium cocoamphodiacetate | 2.5 |
| polysorbate 20 | 2 |
| glycerin | 1.5 |
| PEG-7 glyceryl cocoate | 1.4 |
| disodium ricinoleamido MEA-sulfosuccinate | 1 |
| Example 5 | 1 |
| polyquaternium-10 | 0.6 |
| Parfum | Parfum |
| Preservative | As needed |
| citric acid | As needed, pH 4.5 - 6.5 |
| sodium hydroxide | As needed, pH 4.5 - 6.5 |
| Colorant | As needed |

**Example 80: A shampoo composition**

| Ingredient | Active level, % |
|---|---|
| Aqua | To 100 |
| sodium cocoyl isethionate | 4.5 |
| sodium lauryl sulfoacetate | 3 |
| disodium laureth sulfosuccinate | 2.5 |
| cocamidopropyl betaine | 2.5 |
| sodium lauroyl sarcosinate | 2.3 |
| glycol distearate | 2 |
| glycereth-26 | 1.8 |
| decyl glucoside | 1.7 |
| hydrogenated coconut acid | 1.5 |
| PPG-5-ceteth-20 | 1.5 |
| PEG-55 propylene glycol oleate | 1.5 |
| propylene glycol | 1.5 |
| sodium chloride | 1.5 |
| divinyldimethicone/dimethicone copolymer | 1 |
| polyquaternium-7 | 1 |
| amodimethicone | 1 |
| Example 9 | 1 |
| polyquaternium-10 | 0.8 |
| sodium isethionate | 0.6 |
| carbomer | 0.6 |
| C11-15 pareth-7 | 0.4 |
| glycerin | 0.3 |
| laureth-9 | 0.2 |
| Hibiscus esculentus | 0.1 |
| Parfum | Parfum |
| Preservative | As needed |
| citric acid | As needed, pH 4.5 - 6.5 |
| sodium hydroxide | As needed, pH 4.5 - 6.5 |
| Colorant | As needed |

**Example 81: A shampoo composition**

| Ingredient | Active level, % |
|---|---|
| Aqua | To 100 |
| cocamidopropyl betaine | 5 |
| sodium lauryl sulfoacetate | 4 |
| disodium laureth sulfosuccinate | 3.5 |
| sodium lauroyl sarcosinate | 3 |
| glycol distearate | 2 |
| sodium chloride | 1.7 |
| decyl glucoside | 1.5 |
| Example 19 | 1.5 |
| polyquaternium-10 | 1 |
| PPG-5-ceteth-20 | 1 |
| coco-betaine | 0.8 |
| PEG-55 propylene glycol oleate | 0.8 |
| propylene glycol | 0.5 |
| salicylic acid | 0.4 |
| carbomer | 0.4 |
| Parfum | Parfum |
| Preservative | As needed |
| citric acid | As needed, pH 4.5 - 6.5 |
| sodium hydroxide | As needed, pH 4.5 - 6.5 |
| Colorant | As needed |

**Example 82: A shampoo composition**

| Ingredient | Active level, % |
|---|---|
| Aqua | To 100 |
| sodium laureth sulfate | 10 |
| PEG-200 hydrogenated glyceryl palmate | 3 |
| disodium cocoamphodiacetate | 2.5 |
| polysorbate-20 | 2 |
| sodium chloride | 1.7 |
| Example 6 | 1 |
| glycerin | 1 |
| PEG-7 glyceryl cocoate | 1 |
| polysorbate 21 | 0.5 |
| polyquaternium-10 | 0.2 |
| disodium EDTA | 0.2 |
| Parfum | Parfum |
| Preservative | As needed |
| citric acid | As needed, pH 4.5 - 6.5 |
| sodium hydroxide | As needed, pH 4.5 - 6.5 |
| Colorant | As needed |

**Example 83: A shampoo composition**

| Ingredient | Active level, % |
|---|---|
| Aqua | To 100 |
| sodium laureth sulfate | 15 |
| coco-betaine | 3 |
| glycol distearate | 2.5 |
| sodium chloride | 2 |
| PPG-5-ceteth-20 | 1.8 |
| Example 13 | 1.5 |
| piroctone olamine | 1.5 |
| octyldodecanol | 1 |
| carbomer | 1 |
| guar hydroxypropyltrimonium chloride | 1 |
| sodium hyaluronate | 0.4 |
| Parfum | Parfum |
| Preservative | As needed |
| citric acid | As needed, pH 4.5 - 6.5 |
| sodium hydroxide | As needed, pH 4.5 - 6.5 |
| Colorant | As needed |

**Example 84: A shampoo composition**

| Ingredient | Active level, % |
|---|---|
| Aqua | To 100 |
| sodium laureth sulfate | 16 |
| dimethicone | 3 |
| coco-betaine | 1.5 |
| glycol distearate | 1.5 |
| sodium chloride | 1.5 |
| guar hydroxypropyltrimonium chloride | 1 |
| cocamide MIPA | 1 |
| Example 8 | 1 |
| sodium cocoate | 0.8 |
| benzophenone-4 | 0.7 |
| carbomer | 0.6 |
| Parfum | Parfum |
| Preservative | As needed |
| citric acid | As needed, pH 4.5 - 6.5 |
| sodium hydroxide | As needed, pH 4.5 - 6.5 |
| Colorant | As needed |

**Example 85: A shampoo composition**

| Ingredient | Active level, % |
|---|---|
| Aqua | To 100 |
| sodium laureth sulfate | 12 |
| coco-glucoside | 4 |
| sodium chloride | 2 |
| distearyl ether | 1.5 |
| behenyl alcohol | 1.5 |
| PPG-5-ceteth-20 | 1 |
| aminomethyl propanol | 1 |
| Example 8 | 1 |
| polysilicone-8 | 0.8 |
| polyquaternium-16 | 0.8 |
| carbomer | 0.5 |
| Parfum | Parfum |
| Preservative | As needed |
| citric acid | As needed, pH 4.5 - 6.5 |
| sodium hydroxide | As needed, pH 4.5 - 6.5 |
| Colorant | As needed |

### EXPERIMENTAL

### Testing of the shampoo compositions

The studies are conducted with hair swatches (using straight European hair tresses from Kerling, DE, 15 cm long, ca. 2.6 g hair each). Chemically treated (4 times bleached) hair are used. Two shampoo products were tested: the hair was washed in a commercially available silicone-free shampoo (Balea Jeden Tag Shampoo, Cool Blossom), and the same shampoo to which 2% (active level) of the material of Example 5 was added. Hair swatches were pre-washed two times in tap water (Frankfurt, Germany) with controlled temperature (37°C) and water flow (4 L/min) using a 14% (active ingredient) sodium laureth sulfate, SLES, solution in water (pH 5.5), using 0.1 g/g SLES/Hair. Next, shampoo wash was performed two times, by applying the shampoo, massaging and rinsing the hair with water. Wet detangling force was measured on wet swatches using Diastron (UK) MTT 175 tester, and then the hair was allowed to dry overnight at controlled temperature and humidity (22°C, 45% RH). The following day, hair gloss on the dry swatches was measured with Samba Hair System (Bossa Nova Tech, CA, USA). BNT numbers for hair gloss calculated by the Bossa Nova algorithm are reported. For each product, three hair tresses were used, and each was measured three times.

The following results were obtained:

| Product | Wet detangling force (average), gmf | Hair gloss, BNT |
|---|---|---|
| Balea shampoo | 77 | 6.7 |
| Balea shampoo + 2.0% Example 5 | 49 | 7.0 |

It is evident that the addition of OAS of Example 5 considerably improves the ease of detangling by lowering the force needed to detangle the hair, which is normally perceived by consumers as conditioning and repairing effect. In addition, the OAS of Example 5 increases hair gloss by a level visually perceived by consumers. This gives the effect of repaired and healthier hair. With the conditioning properties, OAS are expected to deliver additional consumer benefits, such as volume and/ or frizz reduction, conditioned and/ or moisturized after-feel on dry hair, ease of styling, etc.

### ANALYTICS

The molecular mass Mn (number average) aka number average molecule weight used herein is measured by GPC. GPC is a special type of liquid chromatography in which the sample is separated according to the hydrodynamic volumes of the individual constituents. Detection is effected by e.g. refractive index and yields a simple distribution curve. To attribute actual molecular weight values to the curve, it is necessary to calibrate the column by passing down polymers of known molecular weight. GPC herein was determined under the following conditions:
Column:
   1x PSS SDV Guard, 5 micron, 50 mm x 8.0 mm ID
   1 x PSS SDV 100 Angstroms, 5 micron, 300 mm x 8.0 mm ID
   1 x PSS SDV 1000 Angstroms, 5 micron, 300 mm x 8.0 mm ID
   1 x PSS SDV 100000 Angstroms, 5 micron, 300 mm x 8.0 mm ID
Detector: RID (refractive index detector)
Oven temperature: 40 °C
Flow: 1 ml/min
Injection volume: 50 µl
Eluent: THF
Calibration method: Conventional
Standards: Polyethylene glycol standards in the range from 430 to 44700 Dalton Internal Standard: Toluene.

## Claims

1. A cosmetic composition, in particular hair conditioner composition, comprising at least one oligoester ammonium salt (OAS) and at least one further cosmetically acceptable component (F), whereby the at least one oligoester ammonium salt (OAS) is obtainable by the following steps:
(i) heating a mixture of the following components (a) to (d) under continuously removing of reaction water:
0.5 to 3.0 molar equivalents of a diethanolamine compound represented by the formula (a) 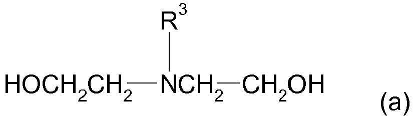 wherein R³ is a linear or branched C₁-C₆-alkyl, preferably linear or branched C₁-C₄-alkyl, more preferably methyl or ethyl;
0.5 to 1.5 molar equivalents of a dicarboxylic acid represented by the formula (b) 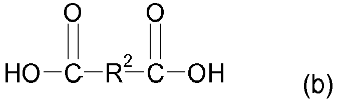 wherein R² is a linear or branched C₁-C₁₀-alkylene or a linear or branched C₂-C₁₀-alkenylene, preferably C₂-C₈-alkylene, more preferably C₄-alkylene;
0.5 to 1.5 molar equivalents of an organic triol represented by the formula (c) 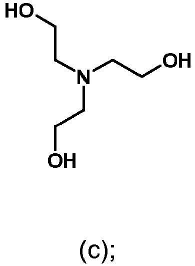 1.0 molar equivalent of a monocarboxylic acid represented by formula (d)
R¹-COOH (d)
wherein R¹ is linear or branched C₁₂-C₂₄-alkyl or linear or branched C₁₂-C₂₄-alkenyl, preferably linear or branched C₁₂-C₂₂-alkyl or linear or branched C₁₂-C₂₂-alkenyl, more preferably C₂₀-C₂₂-alkyl;
(ii) reacting the oligoester product of step (i) with a quaternization agent (e), in particular from the group of dimethyl sulfate, diethyl sulfate and alkyl halides;
(iii) optionally removing and purifying the at least one oligoester ammonium salt (OAS).

2. The cosmetic composition comprising at least one oligoester ammonium salt (OAS) according to claim 1, in which in the diethanolamine component (a), R³ is methyl.

3. The cosmetic composition comprising at least one oligoester ammonium salt (OAS) according to claim 1 or 2, in which the dicarboxylic acid (b) is selected from the group consisting of adipic acid, glutaric acid, succinic acid, sebacid acid, itaconic acid, maleic acid, and combinations thereof.

4. The cosmetic composition comprising at least one oligoester ammonium salt (OAS) according to any one of the claims 1 to 3, wherein the composition is a hair shampoo.

5. The cosmetic composition comprising at least one oligoester ammonium salt (OAS) according to any one of the claims 1 to 4, in which the carboxylic acid (d) is selected from the group consisting of behenic acid, oleic acid, coconut fatty acid, linoleic acid, and combinations thereof.

6. The cosmetic composition comprising at least one oligoester ammonium salt (OAS) according to any one of the claims 1 to 5, in which the components (a), (b), (c) and (d) are used in the following molar ratios: from 0.5 - 3.0, from 0.5 - 1.5, from 0.5 - 1.5, and 1, respectively.

7. The cosmetic composition comprising at least one oligoester ammonium salt (OAS) according to any one of the claims 1 to 6, obtained by first heating the mixture of components (a), (b), (c) and (d) in step (i) to a temperature from 80 to 220 °C, preferably from 150 to 210 °C, more preferably from 160 to 200 °C, and in step (ii) subsequent reaction of the obtained oligoester product with a quarternization agent (e), preferably from the group of dimethyl sulfate, diethyl sulfate, methylchloride, ethylchloride, butylchloride, or combinations thereof.

8. The cosmetic composition comprising at least one oligoester ammonium salt (OAS) according to any one of the claims 1 to 7, in which the oligoester ammonium salt (OAS) has a molecular mass Mn (number average) of from 600 to 4000 g/mol, preferably from 650 to 2000 g/mol, more preferably from 800 to 1900 g/mol, even more preferably from 665 to 1800 g/mol, even more preferably from 900 to 1800 g/mol..

9. A cosmetic composition, in particular hair conditioner composition, comprising at least one oligoester ammonium salt (OAS) of formula (I) and at least one further cosmetically acceptable component (F), 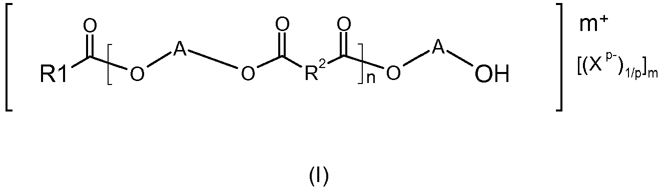 wherein:
R¹ is a linear or branched C₂₀-C₂₄-alkyl or linear or branched C₂₀-C₂₄-alkenyl group;
R² is a linear or branched C₁-C₈-alkylene or linear or branched C₂-C₈-alkenylene group;
A is, at least once, independently selected from quaternary ammonium compounds of the formula (II) 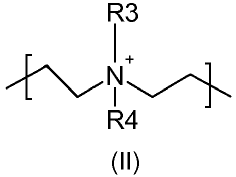 wherein R³, R⁴ are each independently linear or branched C₁-C₆-alkyl or linear or branched hydroxy-C₁-C₆-alkyl groups;
and A is also, at least once, independently selected from quaternary ammonium compounds of the formula (III) 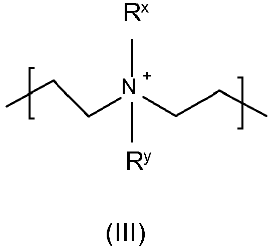 wherein R^{x} is a linear or branched hydroxy-C₁-C₆-alkyl group and R^{y} is a linear or branched C₁-C₆-alkyl;
n is an integer from 1 to 30, preferably 1 to 10; and
m is an integer from 1 to 31, preferably 1 to 10, describing the charge;
[(X^{p-})_{1/p}] is a counter-ion selected from the group consisting of monovalent anions having the formula [X⁻], divalent anions having the formula [(X²⁻)₀.₅], and trivalent anions having the formula [(X³⁻)_{1/3}].

10. The cosmetic composition according to claim 9, **characterized in that** in formula (I) the counter ion of the formula [(X^{p-})_{1/p}] is selected from the group consisting of chloride, bromide, methyl sulfate, ethyl sulfate, formate, citrate, acetate, nitrate, carbonate, sulfate, phosphate, and combinations thereof.

11. The cosmetic composition according to any one of claims 1 to 10, comprising 0.01 to 20 % by weight of one or more oligoester ammonium salts (OAS) and further comprising at least 0.5 % by weight of one or more further components (F) selected from the group consisting of acidity regulators, glossers, lubricants, and further surfactants.

12. The cosmetic composition according to any one of claims 1 to 11, wherein the cosmetic composition is a shampoo composition or a conditioning composition.

13. The shampoo composition according to claim 12, wherein component (F) is an anionic surfactant.

14. The shampoo composition according to any of claims 12 or 13, wherein the composition further comprises a conditioning agent.

15. Use of an oligoester ammonium salt (OAS) obtained by a process as defined in any one of claims 1 to 8, or of an oligoester ammonium salt (OAS) of claims 9 to 14 as a surfactant in cosmetic compositions, preferably in hair conditioner compositions.

16. A method of preparing a cosmetic composition, in particular hair conditioner composition, according to any one of claims 1 to 14, comprising the step of preparing one or more oligoester ammonium salts (OAS) as described in any one of claims 1 to 14, and mixing the OAS with one or more further components (F).

17. A method of treating hair, comprising:
a) applying the hair conditioner composition according to any one of claims 1 to 14 onto wet hair and then
b) removing the conditioner composition from the hair.

18. The method according to claim 17, comprising:
(a) applying a shampoo composition onto the hair; and then
(b) washing the hair with the shampoo composition; and then
(c) removing the shampoo composition from the hair; and then
(d) applying the hair conditioner composition onto wet hair, and then removing the conditioner composition from the hair.

## Patentansprüche

1. Kosmetische Zusammensetzung, insbesondere Haarconditionerzusammensetzung, umfassend mindestens ein Oligoester-Ammoniumsalz (OAS) und mindestens eine weitere kosmetisch unbedenkliche Komponente (F), wobei das mindestens eine Oligoester-Ammoniumsalz (OAS) durch die folgenden Schritte erhältlich ist:
(i) Erhitzen einer Mischung der folgenden Komponenten (a) bis (d) unter kontinuierlicher Entfernung von Reaktionswasser:
0,5 bis 3,0 Moläquivalente einer durch die Formel (a) wiedergegebenen Diethanolaminverbindung: 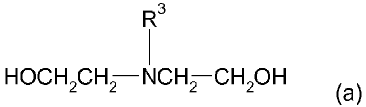 wobei R³ für ein lineares oder verzweigtes C₁-C₆-Alkyl, vorzugsweise lineares oder verzweigtes C₁-C₄-Alkyl, weiter bevorzugt Methyl oder Ethyl, steht;
0,5 bis 1,5 Moläquivalente einer durch die Formel (b) wiedergegebenen Dicarbonsäure: 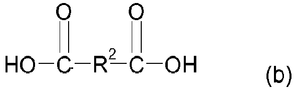 wobei R² für ein lineares oder verzweigtes C₁-C₁₀-Alkylen oder ein lineares oder verzweigtes C₂-C₁₀-Alkenylen, vorzugsweise C₂-C₈-Alkenylen, weiter bevorzugt C₄-Alkenylen, steht;
0,5 bis 1,5 Moläquivalente eines durch die Formel (c) wiedergegebenen organischen Triols: 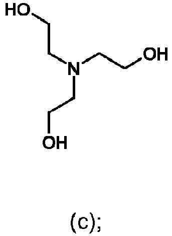
1,0 Moläquivalente einer durch Formel (d) wiedergegebenen Monocarbonsäure:
R¹-COOH (d)
wobei R¹ für lineares oder verzweigtes C₁₂-C₂₄-Alkyl oder lineares oder verzweigtes C₁₂-C₂₄-Alkenyl, vorzugsweise lineares oder verzweigtes C₁₂-C₂₂-Alkyl oder lineares oder verzweigtes C₁₂-C₂₂-Alkenyl, weiter bevorzugt C₂₀-C₂₂-Alkyl, steht;
(ii) Umsetzen des Oligoester-Produkts aus Schritt (i) mit einem Quaternisierungsmittel (e), insbesondere aus der Gruppe Dimethylsulfat, Diethylsulfat und Alkylhalogenide;
(iii) gegebenenfalls Entfernen und Reinigen des mindestens einen Oligoester-Ammoniumsalzes (OAS).

2. Kosmetische Zusammensetzung, umfassend mindestens ein Oligoester-Ammoniumsalz (OAS), nach Anspruch 1, wobei in der Diethanolamin-Komponente (a) R³ für Methyl steht.

3. Kosmetische Zusammensetzung, umfassend mindestens ein Oligoester-Ammoniumsalz (OAS), nach Anspruch 1 oder 2, wobei die Dicarbonsäure (b) aus der Gruppe bestehend aus Adipinsäure, Glutarsäure, Bernsteinsäure, Sebacinsäure, Itaconsäure, Maleinsäure und Kombinationen davon ausgewählt ist.

4. Kosmetische Zusammensetzung, umfassend mindestens ein Oligoester-Ammoniumsalz (OAS), nach einem der Ansprüche 1 bis 3, wobei es sich bei der Zusammensetzung um ein Haarshampoo handelt.

5. Kosmetische Zusammensetzung, umfassend mindestens ein Oligoester-Ammoniumsalz (OAS), nach einem der Ansprüche 1 bis 4, wobei die Carbonsäure (d) aus der Gruppe bestehend aus Behensäure, Ölsäure, Kokosfettsäure, Linolsäure und Kombinationen davon ausgewählt ist.

6. Kosmetische Zusammensetzung, umfassend mindestens ein Oligoester-Ammoniumsalz (OAS), nach einem der Ansprüche 1 bis 5, wobei die Komponenten (a), (b), (c) und (d) in den folgenden Molverhältnissen verwendet werden: von 0,5-3,0, von 0,5-1,5, von 0,5-1,5 bzw. 1.

7. Kosmetische Zusammensetzung, umfassend mindestens ein Oligoester-Ammoniumsalz (OAS), nach einem der Ansprüche 1 bis 6, dadurch erhalten, dass man zuerst die Mischung der Komponenten (a), (b), (c) und (d) in Schritt (i) auf eine Temperatur von 80 bis 220 °C, vorzugsweise von 150 bis 210 °C, weiter bevorzugt 160 bis 200 °C, erhitzt und in Schritt (ii) anschließend das erhaltene Oligoester-Produkt mit einem Quaternisierungsmittel (e), vorzugsweise aus der Gruppe Dimethylsulfat, Diethylsulfat, Methylchlorid, Ethylchlorid, Butylchlorid oder Kombinationen davon, umsetzt.

8. Kosmetische Zusammensetzung, umfassend mindestens ein Oligoester-Ammoniumsalz (OAS), nach einem der Ansprüche 1 bis 7, wobei das Oligoester-Ammoniumsalz (OAS) eine Molmasse Mn (Zahlenmittel) von 600 bis 4000 g/mol, vorzugsweise von 650 bis 2000 g/mol, weiter bevorzugt von 800 bis 1900 g/mol, noch weiter bevorzugt von 665 bis 1800 g/mol, noch weiter bevorzugt von 900 bis 1800 g/mol, aufweist.

9. Kosmetische Zusammensetzung, insbesondere Haarconditionerzusammensetzung, umfassend mindestens ein Oligoester-Ammoniumsalz (OAS) der Formel (I) und mindestens eine weitere kosmetisch unbedenkliche Komponente (F), 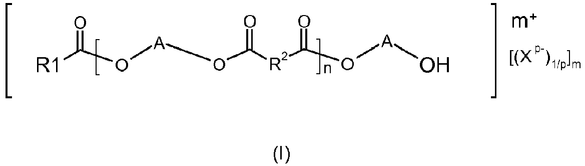 wobei:
R¹ für eine lineare oder verzweigte C₂₀-C₂₄-Alkyl- oder lineare oder verzweigte C₂₀-C₂₄-Alkenylgruppe steht;
R² für eine lineare oder verzweigte C₁-C₈-Alkylen- oder lineare oder verzweigte C₂-C₈-Alkenylengruppe steht;
A mindestens einmal unabhängig aus quaternären Ammoniumverbindungen der Formel (II) 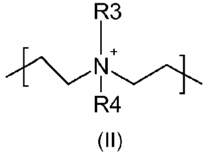 ausgewählt ist, wobei R³ und R⁴ jeweils unabhängig für lineare oder verzweigte C₁-C₆-Alkyl- oder lineare oder verzweigte Hydroxy-C₁-C₆-alkylgruppen stehen;
und A außerdem mindestens einmal unabhängig aus quaternären Ammoniumverbindungen der Formel (III) 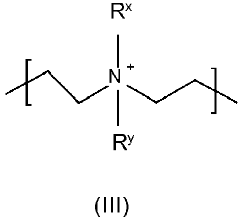 ausgewählt ist, wobei R^{x} für eine lineare oder verzweigte Hydroxy-C₁-C₆-alkylgruppe steht und R^{y} für ein lineares oder verzweigtes C₁-C₆-Alkyl steht;
n für eine ganze Zahl von 1 bis 30, vorzugsweise 1 bis 10, steht und
m für eine ganze Zahl von 1 bis 31, vorzugsweise 1 bis 10, die die Ladung beschreibt, steht;
[(X^{p-})_{1/p}] für ein Gegenion aus der Gruppe bestehend aus einwertigen Anionen mit der Formel [X⁻], zweiwertigen Anionen mit der Formel [(X²⁻)₀,₅] und dreiwertigen Anionen mit der Formel [(X³⁻)_{1/3}] steht.

10. Kosmetische Zusammensetzung nach Anspruch 9, **dadurch gekennzeichnet, dass** in Formel (I) das Gegenion der Formel [(X^{p-})_{1/p}] aus der Gruppe bestehend aus Chlorid, Bromid, Methylsulfat, Ethylsulfat, Formiat, Citrat, Acetat, Nitrat, Carbonat, Sulfat, Phosphat und Kombinationen davon ausgewählt ist.

11. Kosmetische Zusammensetzung nach einem der Ansprüche 1 bis 10, umfassend 0,01 bis 20 Gew.-% eines oder mehrerer Oligoester-Ammoniumsalze (OAS) und ferner umfassend mindestens 0,5 Gew.-% einer oder mehrerer weiterer Komponenten (F) aus der Gruppe bestehend aus Säureregulatoren, Glanzmitteln, Gleitmitteln und weiteren Tensiden.

12. Kosmetische Zusammensetzung nach einem der Ansprüche 1 bis 11, wobei es sich bei der kosmetischen Zusammensetzung um eine Shampoozusammensetzung oder eine Conditionerzusammensetzung handelt.

13. Shampoozusammensetzung nach Anspruch 12, wobei es sich bei Komponente (F) um ein anionisches Tensid handelt.

14. Shampoozusammensetzung nach einem der Ansprüche 12 oder 13, wobei die Zusammensetzung ferner ein Konditionierungsmittel umfasst.

15. Verwendung eines durch ein Verfahren gemäß einem der Ansprüche 1 bis 8 erhaltenen Oligoester-Ammoniumsalzes (OAS) oder eines Oligoester-Ammoniumsalzes (OAS) der Ansprüche 9 bis 14 als Tensid in kosmetischen Zusammensetzungen, vorzugsweise in Haarconditionerzusammensetzungen.

16. Verfahren zur Herstellung einer kosmetischen Zusammensetzung, insbesondere einer Haarconditionerzusammensetzung, nach einem der Ansprüche 1 bis 14, umfassend den Schritt des Herstellens eines oder mehrerer Oligoester-Ammoniumsalze (OAS) wie in einem der Ansprüche 1 bis 14 beschrieben und des Mischens des OAS mit einer oder mehreren weiteren Komponenten (F).

17. Verfahren zum Behandeln von Haar, das Folgendes umfasst:
a) Aufbringen der Haarconditionerzusammensetzung nach einem der Ansprüche 1 bis 14 auf nasses Haar und dann
b) Entfernen der Conditionerzusammensetzung aus dem Haar.

18. Verfahren nach Anspruch 17, umfassend:
(a) Aufbringen einer Shampoozusammensetzung auf das Haar und dann
(b) Waschen des Haars mit der Shampoozusammensetzung und dann
(c) Entfernen der Shampoozusammensetzung aus dem Haar und dann
(d) Aufbringen der Haarconditionerzusammensetzung auf nasses Haar und dann Entfernen der Conditionerzusammensetzung aus dem Haar.

## Revendications

1. Composition cosmétique, en particulier composition d'après-shampooing, comprenant au moins un sel d'ammonium d'oligoester (OAS) et au moins un composant acceptable sur le plan cosmétique supplémentaire (F), l'au moins un sel d'ammonium d'oligoester (OAS) pouvant être obtenu par les étapes suivantes :
(i) chauffage d'un mélange des composants suivants (a) à (d) sous élimination de manière continue de l'eau de réaction :
0,5 à 3,0 équivalents molaires d'un composé de type diéthanolamine représenté par la formule (a) 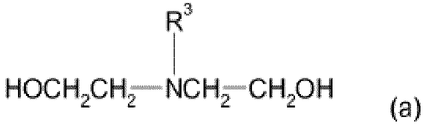 R³ étant un C₁₋₆-alkyle linéaire ou ramifié, préférablement C₁₋₄-alkyle linéaire ou ramifié, plus préférablement méthyle ou éthyle ;
0,5 à 1,5 équivalents molaires d'un acide dicarboxylique représenté par la formule (b) 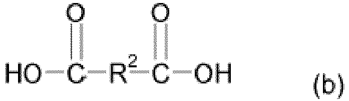 R² étant un C₁₋₁₀-alkylène linéaire ou ramifié ou un C₂₋₁₀-alcénylène linéaire ou ramifié, préférablement C₂₋₈-alcénylène, plus préférablement C₄-alcénylène ;
0,5 à 1,5 équivalents molaires d'un triol organique représenté par la formule (c) 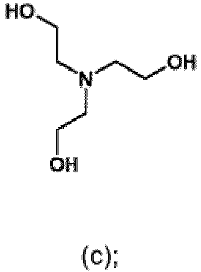
1,0 équivalent molaire d'un acide monocarboxylique représenté par la formule (d)
R¹-COOH (d)
R¹ étant un C₁₂₋₂₄-alkyle linéaire ou ramifié ou un C₁₂₋₂₄-alcényle linéaire ou ramifié, préférablement C₁₂₋₂₂-alkyle linéaire ou ramifié ou un C₁₂₋₂₂-alcényle linéaire ou ramifié, plus préférablement C₂₀₋₂₂-alkyle ;
(ii) mise en réaction du produit de type oligoester de l'étape (i) avec un agent de quaternisation (e), en particulier du groupe composé par le sulfate de diméthyle, le sulfate de diéthyle et des halogénures d'alkyle ;
(iii) éventuellement, élimination et purification de l'au moins un sel d'ammonium d'oligoester (OAS).

2. Composition cosmétique comprenant au moins un sel d'ammonium d'oligoester (OAS) selon la revendication 1, dans laquelle dans le composant de type diéthanolamine (a), R³ est méthyle.

3. Composition cosmétique comprenant au moins un sel d'ammonium d'oligoester (OAS) selon la revendication 1 ou 2, dans laquelle l'acide dicarboxylique (b) est choisi dans le groupe constitué par l'acide adipique, l'acide glutarique, l'acide succinique, l'acide sébacique, l'acide itaconique, l'acide maléique, et des combinaisons correspondantes.

4. Composition cosmétique comprenant au moins un sel d'ammonium d'oligoester (OAS) selon l'une quelconque des revendications 1 à 3, la composition étant un shampooing capillaire.

5. Composition cosmétique comprenant au moins un sel d'ammonium d'oligoester (OAS) selon l'une quelconque des revendications 1 à 4, dans laquelle l'acide carboxylique (d) est choisi dans le groupe constitué par l'acide béhénique, l'acide oléique, un acide gras de noix de coco, l'acide linoléique, et des combinaisons correspondantes.

6. Composition cosmétique comprenant au moins un sel d'ammonium d'oligoester (OAS) selon l'une quelconque des revendications 1 à 5, dans laquelle les composants (a), (b), (c) et (d) sont utilisés dans les rapports molaires suivants : respectivement de 0,5 à 3,0, de 0,5 à 1,5, de 0,5 à 1,5, et 1.

7. Composition cosmétique comprenant au moins un sel d'ammonium d'oligoester (OAS) selon l'une quelconque des revendications 1 à 6, obtenue en mélangeant d'abord le mélange de composants (a), (b), (c) et (d) dans l'étape (i) à une température de 80 à 220 °C, préférablement de 150 à 210 °C, plus préférablement de 160 à 200 °C, et dans l'étape (ii) réaction subséquente du produit de type oligoester obtenu avec un agent de quaternisation (e), préférablement du groupe composé par le sulfate de diméthyle, le sulfate de diéthyle, le chlorure de méthyle, le chlorure d'éthyle, le chlorure de butyle ou des combinaisons correspondantes.

8. Composition cosmétique comprenant au moins un sel d'ammonium d'oligoester (OAS) selon l'une quelconque des revendications 1 à 7, dans laquelle le sel d'ammonium d'oligoester (OAS) possède une masse moléculaire Mn (moyenne en nombre) allant de 600 à 4 000 g/mole, préférablement de 650 à 2 000 g/mole, plus préférablement de 800 à 1 900 g/mole, encore plus préférablement de 665 à 1 800 g/mole, encore plus préférablement de 900 à 1 800 g/mole.

9. Composition cosmétique, en particulier composition d'après-shampooing, comprenant au moins un sel d'ammonium d'oligoester (OAS) de formule (I) et au moins un composant acceptable sur le plan cosmétique supplémentaire (F), 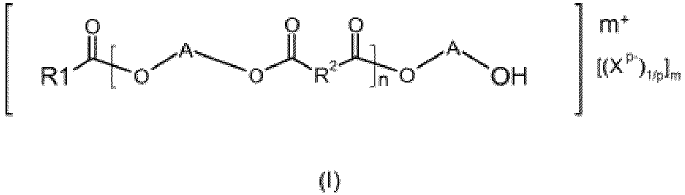
R¹ étant un groupe C₂₀₋₂₄-alkyle linéaire ou ramifié ou un C₂₀₋₂₄-alcényle linéaire ou ramifié ;
R² étant un groupe C₁₋₈-alkylène linéaire ou ramifié ou un C₂₋₈-alcénylène linéaire ou ramifié ;
A étant, au moins une fois, indépendamment choisi parmi des composés de type ammonium quaternaire de la formule (II) 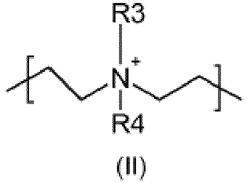
R³, R⁴ étant chacun indépendamment des groupes C₁₋₆-alkyle linéaires ou ramifiés ou hydroxy-C₁₋₆-alkyle linéaires ou ramifiés ;
et A étant également, au moins une fois, indépendamment choisi parmi des composés de type ammonium quaternaire de la formule (III) 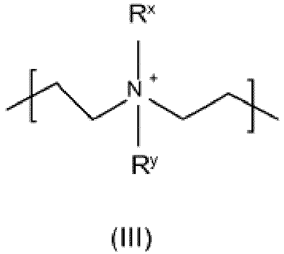
R^{x} étant un groupe hydroxy-C₁₋₆-alkyle linéaire ou ramifié et R^{y} étant un C₁₋₆-alkyle linéaire ou ramifié ;
n étant un entier de 1 à 30, préférablement de 1 à 10 ; et
m étant un entier de 1 à 31, préférablement de 1 à 10, décrivant la charge ;
[(X^{p-})_{1/p}] étant un contre-ion choisi dans le groupe constitué par des anions monovalents possédant la formule [X⁻], des anions divalents possédant la formule [(X²⁻)_{0,5}], et des anions trivalents possédant la formule [(X³⁻)_{1/3}].

10. Composition cosmétique selon la revendication 9, **caractérisée en ce que** dans la formule (I) le contre-ion de la formule [(X^{p-})_{1/p}] est choisi dans le groupe constitué par chlorure, bromure, méthylsulfate, éthylsulfate, formiate, citrate, acétate, nitrate, carbonate, sulfate, phosphate, et des combinaisons correspondantes.

11. Composition cosmétique selon l'une quelconque des revendication 1 à 10, comprenant 0,01 à 20 % en poids d'un ou plusieurs sels d'ammonium d'oligoester (OAS) et comprenant en outre au moins 0,5 % en poids d'un ou plusieurs composants supplémentaires (F) choisis dans le groupe constitué par des régulateurs d'acidité, des agents de brillance, des lubrifiants et d'autres tensioactifs.

12. Composition cosmétique selon l'une quelconque des revendications 1 à 11, la composition cosmétique étant une composition de shampooing ou une composition d'après-shampooing.

13. Composition de shampooing selon la revendication 12, le composant (F) étant un tensioactif anionique.

14. Composition de shampooing selon l'une quelconque des revendications 12 et 13, la composition comprenant en outre un agent de conditionnement.

15. Utilisation d'un sel d'ammonium d'oligoester (OAS) obtenu par un procédé tel que défini dans l'une quelconque des revendications 1 à 8, ou d'un sel d'ammonium d'oligoester (OAS) selon les revendications 9 à 14 en tant que tensioactifs dans des compositions cosmétiques, préférablement dans des compositions d'après-shampooing.

16. Procédé de préparation d'une composition cosmétique, en particulier d'une composition d'après-shampooing, selon l'une quelconque des revendications 1 à 14, comprenant l'étape de préparation d'un ou plusieurs sels d'ammonium d'oligoester (OAS) tels que décrits dans l'une quelconque des revendications 1 à 14, et le mélange de l'OAS avec un ou plusieurs composants supplémentaires (F).

17. Procédé de traitement de cheveux, comprenant :
a) l'application de la composition d'après-shampooing selon l'une quelconque des revendications 1 à 14 sur des cheveux mouillés et ensuite
b) l'élimination de la composition d'après-shampooing des cheveux.

18. Procédé selon la revendication 17, comprenant :
(a) l'application d'une composition de shampooing sur les cheveux ; et ensuite
(b) le lavage des cheveux avec la composition de shampooing ; et ensuite
(c) l'élimination de la composition de shampooing des cheveux ; et ensuite
(d) l'application de la composition d'après-shampooing sur les cheveux mouillés, et ensuite l'élimination de la composition d'après-shampooing des cheveux.
